# FASCICULE DE BREVET EUROPEEN

(11) **EP 1 069 132 B1**
(45) Date de publication et mention de la délivrance du brevet: **01.10.2003**
(21) Numéro de dépôt: 00401686.1
(22) Date de dépôt: 15.06.2000
(51) Int. Cl.: C07K 5/078, A61K 38/05, A61P 7/02

(54) **Dérivés bicycliques d'amino-pyrazinones, leur procédé de préparation et les compositions pharmaceutiques qui les contiennent**
Bizyklische Amino-Pyrazinon-Derivate, verfahren zu deren Herstellung und pharmazeutische Zusammensetzungen die diese enthalten
Bicyclic derivatives of amino-pyrazinones, process of preparation and pharmaceutical compositions comprising them

(30) Priorité: 15.06.1999 FR 9907538
(43) Date de publication de la demande: 17.01.2001
(73) Titulaire: LES LABORATOIRES SERVIER, 92200 Neuilly sur Seine (FR)
(72) Inventeur: de Nanteuil, Guillaume, 92150 Suresnes (FR); Gloanec, Philippe, 78380 Bougival (FR); Verbeuren, Tony, 78540 Vernouillet (FR); Rupin, Alain, 37510 Savonnieres (FR); Vallez, Marie-Odile, 77420 Champs sur Marne (FR)

(56) Documents cités:
- WO-A-96/19483
- WO-A-96/30396
- WO-A-97/30708
- WO-A-98/09987
- WO-A-98/17274

## Description

La présente invention concerne des nouveaux dérivés bicycliques d'amino-pyrazinones, leur procédé de préparation, les compositions pharmaceutiques qui les contiennent ainsi que leur utilisation en tant qu'inhibiteurs de protéases à sérine apparentées à la trypsine.

L'une de ces protéases à sérine, la thrombine, est l'enzyme clé de la coagulation et joue un rôle central dans la pathologie des thromboses veineuses et artérielles, en raison notamment de son fort pouvoir d'auto-amplification de la cascade de la coagulation (F. Toti et coll., Sang, Thrombose, Vaisseaux 1992, 4, 483-494 et T.M. Reilly et coll., Blood Coagulation and Fibrinolysis 1992, 3, 513-517).

L'inhibition directe et spécifique de la thrombine est plus efficace et présente moins de risques d'hémorragie que le traitement par l'héparine. Il existe actuellement des inhibiteurs directs de thrombine, mais ces substances peptidiques présentent l'inconvénient de ne pas être actives par voie orale.

Des dérivés peptidomimétiques, présentant une activité anti-thrombotique orale, ont déjà été décrits dans la littérature. C'est le cas notamment des dérivés de l'acide boronique décrits dans les brevets EP 293 881, EP 471 651, EP 615 978 et EP 792 883 et des dérivés décrits dans les brevets WO 94 29336 et WO 95 23609.

Il était donc particulièrement intéressant de synthétiser de nouveaux inhibiteurs de protéases à sérine afin d'augmenter la puissance et la sélectivité des composés déjà décrits dans la littérature.

L'activité de ces nouveaux composés est objectivée par l'augmentation de différents temps de coagulation.

De plus, ces composés sont actifs par voie orale.

Plus spécifiquement, la présente invention concerne les composés de formule (I): 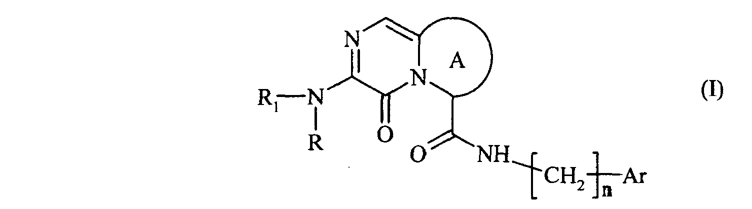 dans laquelle
∗ R₁ représente un atome d'hydrogène ou un groupement alkyle (C₁-C₆) linéaire ou ramifié (éventuellement substitué par un ou plusieurs groupements, identiques ou différents, choisis parmi aryle, hétéroaryle, cycloalkyle, hétérocycloalkyle, carboxy, alkoxycarbonyle (C₁-C₆) linéaire ou ramifié, carbamoyle et hydroxy), ou un groupement cycloalkyle, un groupement hétérocycloalkyle ou un groupement de formule (G) 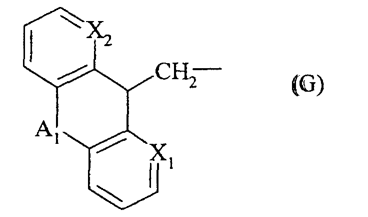 dans laquelle A₁ représente une liaison simple, un groupement -CH₂-, -CH₂-CH₂-, -N(CH₃)- ou un atome d'oxygène ou de soufre, et X₁ et X₂, identiques ou différents, représentent chacun un atome de carbone ou d'azote,
∗ R représente un atome d'hydrogène
∗ 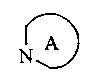 représente un cycle saturé de 4 à 7 chaînons pouvant contenir, en plus de l'atome d'azote, un ou deux hétéroatomes ou groupements choisis parmi O, S, ou -N(R₂),
   - R₂ représente un atome d'hydrogène ou un groupement alkyle (C₁-C₆) linéaire ou ramifié,
∗ n représente un entier tel que 1≤n≤6,
∗ Ar représente un groupement aryle ou hétéroaryle,
leurs isomères, leurs N-oxydes ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, etc.

Parmi les bases pharmaceutiquement acceptables, on peut citer à titrer non limitatif l'hydroxyde de sodium, l'hydroxyde de potassium, la triéthylamine, la tertbutylamine, etc.

Par groupement aryle, on entend phényle, biphénylyle ou naphtyle, chacun de ces groupements étant éventuellement substitué par un ou plusieurs groupements, identiques ou différents, choisis parmi halogène, alkyle (C₁-C₆) linéaire ou ramifié (éventuellement substitué par un groupement hydroxy, carboxy ou carbamoyle (lui-même éventuellement substitué par un ou deux groupements alkyle (C₁-C₆) linéaire ou ramifié)), alkoxy (C₁-C₆) linéaire ou ramifié, hydroxy, trihalogénoalkyle (C₁-C₆) linéaire ou ramifié, trihalogénoalkoxy (C₁-C₆) linéaire ou ramifié, amino (éventuellement substitué par un ou deux groupements alkyle (C₁-C₆) linéaire ou ramifié), alkylcarbonyloxy (C₁-C₆) linéaire ou ramifié, carboxyméthoxy et carbamoylméthoxy (éventuellement N-substitué par un ou deux groupements alkyle (C₁-C₆) linéaire ou ramifié).

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle (C₁-C₆) linéaire ou ramifié (éventuellement substitué par un groupement hydroxy, carboxy ou carbamoyle (lui-même éventuellement substitué par un ou deux groupements alkyle (C₁-C₆) linéaire ou ramifié)), hydroxy, alkoxy (C₁-C₆) linéaire ou ramifié, trihalogénoalkyle (C₁-C₆) linéaire ou ramifié, phényle, amino (éventuellement N-substitué par un ou plusieurs groupements alkyle (C₁-C₆) linéaire ou ramifié), carboxyméthoxy et carbamoylméthoxy (éventuellement N- substitué par un ou deux groupements alkyle (C₁-C₆) linéaire ou ramifié).
Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, pyrimidinyle, pyrazinyle, pyridazinyle.

Par groupement cycloalkyle, on entend un groupement hydrocarboné mono- ou bicyclique, saturé ou insaturé, de 3 à 12 chaînons, étant entendu que le cycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié) et aryle.
Parmi les groupements cycloalkyle, on peut citer à titre non limitatif les groupements cyclopentyle, cyclohexyle, indanyle, tétrahydronaphtyle.

Par groupement hétérocycloalkyle, on entend un groupement mono- ou bicyclique, saturé ou insaturé, de 4 à 12 chaînons contenant un, deux ou trois hétéroatomes choisis parmi oxygène, azote ou soufre, étant entendu que l'hétérocycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié), aryle et diarylméthyle. Parmi les groupements hétérocycloalkyle, on peut citer à titre non limitatif les groupements azétidinyle, pyrrolidinyle, pipéridyle, dihydrocyclopenta[b]pyridyle.

Les composés préférés de formule (I) sont ceux pour lesquels n est égal à 1.

Le cycle 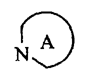 tel que défini dans la formule (I) est préférentiellement un groupement pyrrolidinyle.

Le groupement Ar tel que défini dans la formule (I) est préférentiellement un groupement phényle ou pyridyle, chacun de ces groupements étant éventuellement substitué par un ou plusieurs groupements, identiques ou différents, choisis parmi halogène, alkyle (C₁-C₆) linéaire ou ramifié (éventuellement substitué par un groupement hydroxy, carboxy ou carbamoyle (lui-même éventuellement substitué par un ou deux groupements alkyle (C₁-C₆) linéaire ou ramifié)), alkoxy (C₁-C₆) linéaire ou ramifié, hydroxy, trihalogénoalkyle (C₁-C₆) linéaire ou ramifié, amino (éventuellement substitué par un ou deux groupements alkyle (C₁-C₆) linéaire ou ramifié), carboxyméthoxy et carbamoylméthoxy (éventuellement N-substitué par un ou deux groupements alkyle (C₁-C₆) linéaire ou ramifié).

L'invention s'étend également au procédé de préparation des composés de formule (I) caractérisé en ce que l'on réduit un composé de formule (II) : 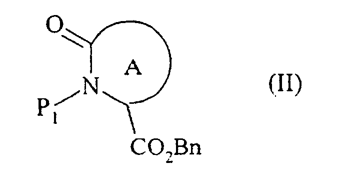 dans laquelle A a la même signification que dans la formule (I), P₁ représente un groupement protecteur de la fonction amino et Bn représente le groupement benzyle,
à l'aide d'un agent réducteur approprié,
pour conduire au composé de formule (III) 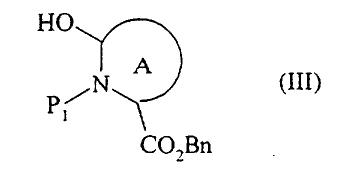 dans laquelle A, P₁ et Bn ont la même signification que précédemment,
composé de formule (III) dont on transforme la fonction hydroxy en méthoxy puis en fonction cyano par des réactions classiques de la chimie organique, pour conduire, après déprotection de la fonction amino, au composé de formule (IV): 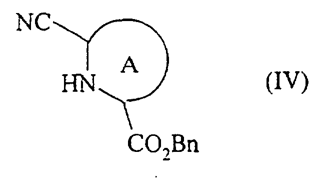 dans laquelle A et Bn ont la même signification que précédemment,
composé de formule (IV) que l'on met en réaction avec du chlorure d'oxalyle pour conduire au composé de formule (V) 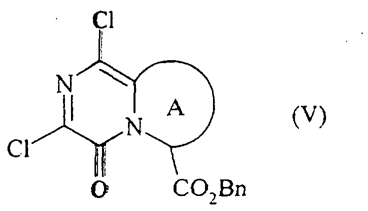 dans laquelle A et Bn ont la même signification que précédemment,
composé de formule (V) que l'on met en réaction avec un composé de formule (VI) :

R₁-NH₂ (VI)

dans laquelle R₁ a la même signification que dans la formule (I), pour conduire au composé de formule (VII) : 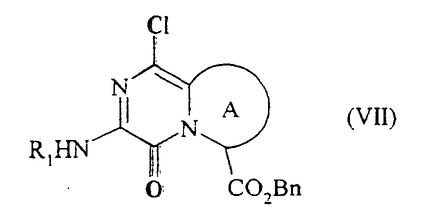 dans laquelle A, Bn et R₁ ont la même signification que précédemment, composé de formule (VII) que l'on transforme ensuite par hydrogénation catalytique en composé de formule (VIII) : 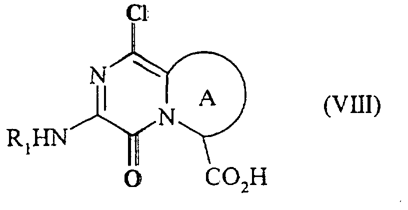 dans laquelle A et R₁ ont la même signification que précédemment,
composé de formule (VIII) que l'on transforme ensuite, par hydrogénation catalytique en milieu alcalin, en composé de formule (IX) : 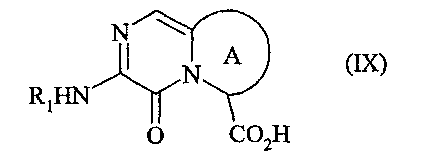 dans laquelle A et R₁ ont la même signification que précédemment,
composé de formule (IX) que l'on met en réaction avec un composé de formule (X) : 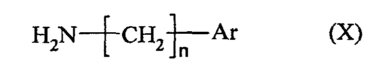 dans laquelle n et Ar ont la même signification que dans la formule (I),
pour conduire, après éventuelle déprotection, au composé de formule (I),
composé de formule (I) que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

Les composés de formule (II) sont obtenus par benzylation des acides correspondants.

Les composés de la présente invention, présentent des propriétés pharmacologiques particulièrement intéressantes.

Ce sont de puissants inhibiteurs de protéases à sérine apparentées à la trypsine qui présentent une importante sélectivité vis-à-vis de la thrombine par rapport à d'autres protéases à sérine de la coagulation et de la fibrinolyse.

Ces propriétés les rendent donc utiles dans le traitement des angines stables ou non, des maladies d'origine thrombotique et/ou donnant lieu à des complications thrombotiques, dans le traitement ou la prévention de l'infarctus du myocarde et des thromboses veineuses ou artérielles, ainsi que dans le traitement des complications des maladies vasculaires et cardiovasculaires telles que l'athérosclérose, l'artérite, la maladie veineuse, et dans le traitement de toutes les maladies impliquant une formation et/ou une activité de la thrombine.

Ils peuvent également être utilisés en association thérapeutique avec un thrombolytique.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention mais ne la limitent en aucune façon.

Par composé de configuration (2α) ou (2β), on entend composé choisi parmi les stéréoisomères (2*R*) et (2*S*), étant entendu que lorsque le composé (2α) représente l'un des stéréoisomères (2*R*) ou (2*S*), alors le composé (2β) représente l'autre stéréoisomère, la configuration absolue du carbone 2 n'étant pas définie.

Les produits de départ utilisés sont des produits de départ connus ou préparés selon des modes opératoires connus.

Les préparations A à G conduisent à des intermédiaires de synthèse utiles pour la préparation des composés de l'invention.

Les structures des composés décrits dans les exemples ont été déterminées selon les techniques spectrophotométriques usuelles (infrarouge, RMN, spectrométrie de masse).

### PREPARATION A: 3-Aminométhyl-6-tert-butyloxycarbonylamino-2-méthylpyridine

### Stade A : 6-Amino-3-cyano-2-méthylpyridine

A 10 mmoles de 6-amino-3-bromo-2-méthylpyridine en solution dans le diméthylformamide est ajouté du cyanure de cuivre (I) (12 mmoles). Le mélange est porté au reflux pendant 10 heures, puis refroidi à 80°C et coulé dans une solution de cyanure de sodium (40 mmoles) dans l'eau. Après 1 heure d'agitation à température ambiante, le mélange est extrait à l'acétate d'éthyle. La phase organique est lavée, puis séchée et évaporée pour conduire au produit attendu sous la forme d'un solide ocre.

### Stade B : 6-Tert-butyloxycarbonylamino-3-cyano-2-méthylpyridine

A 10 mmoles du composé décrit dans le stade précédent en solution dans le tert-butanol est ajoutée une solution de soude IN (11 mmoles) et du dicarbonate de di-tert-butyle (11 mmoles). Après 1 heure d'agitation, les solvants sont évaporés, le résidu est repris par de l'acétate d'éthyle, la phase organique est lavée, séchée et évaporée pour conduire au produit attendu.

### Stade C : 6-Tert-butyloxycarbonylamino-3-aminométhyl-2-méthylpyridine

Une solution du composé décrit dans le stade précédent (10 mmoles) dans l'éthanol est placée sous hydrogène pendant une nuit en présence de nickel de Raney. Après filtration du catalyseur, le solvant est évaporé pour conduire au produit attendu.

### PREPARATION B : 3-Aminométhyl-6-tert-butyloxycarbonylamino-2,5-diméthyl-pyridine

Le produit attendu est obtenu selon le procédé décrit dans la préparation A à partir de 6-amino-3-bromo-2,5-diméthyl-pyridine.

### PREPARATION C : 3-Aminométhyl-6-tert-butyloxycarbonylamino-2,4-diméthyl-pyridine

Le produit attendu est obtenu selon le procédé décrit dans la préparation A à partir de 6-amino-3-bromo-2,4-diméthyl-pyridine.

### PREPARATION D : 3-Aminométhyl-6-tert-butyloxycarbonylamino-2-éthylpyridine

Le produit attendu est obtenu selon le procédé décrit dans la préparation A à partir de 6-amino-3-bromo-2-éthylpyridine.

### PREPARATION E : 2-[2-(Aminométhyl)-phénoxy]-N-éthylacétamide

### Stade A : 2-(2-Cyanophénoxy)-acétate d'éthyle

A une solution de 2-hydroxybenzonitrile (10 mmoles) dans l'acétonitrile est ajouté du carbonate de potassium (30 mmoles) puis du bromo-2-acétamide (11 mmoles). Après une nuit d'agitation, la solution est filtrée et évaporée, le résidu est repris par de l'acétate d'éthyle. la phase organique est lavée, séchée puis évaporée pour conduire au produit attendu.

### Stade B : Acide 2-[2-cyanophénoxy]-acétique

A une solution à 0°C du composé obtenu dans le stade précédent (10 mmoles) dans le tétrahydrofurane est ajoutée de la soude 1N (11 mmoles). Après une nuit d'agitation, le milieu réactionnel est évaporé, le résidu est repris par de l'eau. La phase aqueuse est lavée à l'acétate d'éthyle puis acidifiée par de l'acide chlorhydrique 4N. Le précipité obtenu est filtré puis séché.

### Stade C : 2-[2-Cyanophénoxy]-N-éthylacétamide

A une solution contenant le produit obtenu au stade précédent (10 mmoles) dans le diméthylformamide est ajoutée de la N-hydroxysuccinimide (11 mmoles) et du N,N'-dicyclohexylcarbodiimide (11 mmoles). Après 2 heures d'agitation à température ambiante, l'éthylamine est ajoutée (50 mmoles). Après une nuit d'agitation, le milieu réactionnel est évaporé, filtré puis repris par de l'acétate d'éthyle. La phase organique est lavée à l'eau, séchée puis évaporée pour conduire au produit attendu.

### Stade D : 2-[2-(Aminométhyl)-phénoxy]-N-éthylacétamide

Le produit attendu est obtenu selon le procédé décrit dans le stade C de la préparation A à partir du composé obtenu dans le stade précédent.

### PREPARATION F : 2-[2-(Aminométhyl)-4-chlorophénoxy]-N-éthylacétamide

Le produit attendu est obtenu selon le procédé décrit dans la préparation E en remplaçant le 2-hydroxybenzonitrile par le 5-chloro-2-hydroxybenzonitrile.

### PREPARATION G : 3-Aminométhyl-6-tert-butyloxycarbonylamino-2-hydroxyméthyl-pyridine

Le produit attendu est obtenu selon le procédé décrit dans la préparation A à partir de 6-amino-3-bromo-2-hydroxyméthyl-pyridine.

### EXEMPLE 1 : (6S)-N-[(6-Amino-2-méthyl-3-pyridyl)-méthyl]-4-oxo-3-(2-phénéthylamino)-4,6,7,8-tétrahydropyrrolo[1,2-a]pyrazine-6-carboxamide, dichlorhydrate

### Stade A : (2S)-N-Tert-butoxycarbonyl-5-oxoprolinate de benzyle

A 10 mmoles de (2*S*)-5-oxoprolinate de benzyle (dont le procédé de préparation est décrit par E. Campaigne et coll. (J. Heterocycl. Chem. 1975, 12, 391)) en solution dans le dichlorométhane sont ajoutées à 0°C 11 mmoles de diméthylaminopyridine et 11 mmoles de dicarbonate de di-tert-butyle. Après 24 heures d'agitation à température ambiante, le milieu réactionnel est lavé puis séché et évaporé pour conduire au produit attendu sous la forme d'une huile visqueuse.

### Stade B : (2S)-N-Tert-butoxycarbonyl-5-hydroxy-prolinate de benzyle

A 10 mmoles du composé obtenu dans le stade précédent en solution dans le tétrahydrofurane sont ajoutées, sous argon et à -78°C, 18 mmoles d'une solution 1M d'hydrure de diisobutylaluminium dans l'hexane. Après 20 minutes d'agitation à -78°C, une solution aqueuse saturée de chlorure d'ammonium, puis une solution aqueuse de carbonate de sodium à 10 % sont ajoutées. Après 1 nuit d'agitation à température ambiante, le milieu réactionnel est filtré, le filtrat est évaporé, repris par du dichlorométhane. La phase organique est lavée, séchée puis évaporée. Le résidu est purifié par chromatographie sur gel de silice, en utilisant comme éluant un mélange dichlorométhane/acétate d'éthyle 95/5. On obtient le produit attendu sous la forme d'une huile jaune.

### Stade C : (2S)-N-Tert-butoxycarbonyl-5-méthoxy-prolinate de benzyle

A 10 mmoles du composé obtenu dans le stade précédent est ajoutée une solution d'acide para-toluène sulfonique à 0,1 % dans le méthanol anhydre (88 ml). Après ½ heure d'agitation, une solution aqueuse de carbonate de sodium à 10 % est ajoutée et le produit est extrait par du dichlorométhane. On obtient le produit attendu sous la forme d'une huile légèrement jaune.

### Stade D : (2S)-5-Cyano-prolinate de benzyle, chlorhydrate

A 10 mmoles du composé obtenu dans le stade précédent sont ajoutées, à -40°C et sous argon, une solution de tétrachlorure d'étain dans le dichlorométhane anhydre à 5 % v/v (7,1 ml), puis du cyanure de triméthylsilyle (20,6 mmoles). Après 2 heures d'agitation à -40°C, une solution aqueuse de carbonate de sodium à 10 % est ajoutée, la phase aqueuse est extraite au dichlorométhane, la phase organique est lavée, séchée puis évaporée. Le résidu obtenu est purifié par chromatographie sur gel de silice en utilisant comme éluant un mélange dichlorométhane/acétate /acétate d'éthyle 95/5. L'huile jaune obtenue est mise en solution dans l'acétate d'éthyle puis on fait passer à 0°C un courant d'acide chlorhydrique gaz pendant 30 minutes. Après une nuit d'agitation à température ambiante, le précipité formé est filtré, rincé à l'acétate d'éthyle et séché sous vide au dessicateur.

### Stade E : (6S)-1,3-Dichloro-4-oxo-4,6,7,8-tétrahydropyrrolo[1,2-a]pyrazine-6-carboxylate de benzyle

A 10 mmoles du composé obtenu dans le stade précédent en solution dans l'orthodichlorobenzène est ajouté du chlorure d'oxalyle (40 mmoles). Après 15 heures d'agitation à 100°C, le mélange est ramené à température ambiante et les solvants sont évaporés. Le résidu obtenu est purifié par chromatographie sur gel de silice en utilisant comme éluant un mélange dichlorométhane/acétate d'éthyle 95/5. On obtient le produit attendu sous la forme d'un solide beige.

### Stade F : (6S)-1-Chloro-4-oxo-3-(2-phénéthylamino)-4,6,7,8-tétrahydropyrrolo-[1,2-a]-pyrazine-6-carboxylate de benzyle

A 10 mmoles du composé obtenu dans le stade précédent en solution dans l'acétate d'éthyle sont ajoutées 30 mmoles de 2-phénéthylamine. Après 2 heures d'agitation à reflux, le milieu réactionnel est ramené à température ambiante et de l'acétate d'éthyle est ajouté. La phase organique est lavée, séchée puis évaporée. Le résidu obtenu est purifié par chromatographie sur gel de silice en utilisant comme éluant un mélange dichlorométhane/acétate d'éthyle. On obtient le produit attendu sous la forme d'un solide blanc.

### Stade G : Acide (6S)-1-chloro-4-oxo-3-(2-phénéthylamino)-4,6,7,8-tétrahydropyrrolo[1,2-a]-pyrazine-6-carboxylique

A 10 mmoles du composé obtenu dans le stade précédent en solution dans le dioxane sont ajoutés 42 mg de Pd/C à 10 %. Le mélange est placé sous hydrogène pendant 5 heures à pression et température ambiantes. Après filtration du catalyseur, le solvant est évaporé pour conduire au produit attendu sous la forme d'un solide blanc.

### Stade H : Acide (6S)-4-oxo-3-(2-phénéthylamino)-4,6,7,8-tétrahydropyrrolo[1,2-a]-pyrazine-6-carboxylique

A 10 mmoles du composé obtenu dans le stade précédent en solution dans le dioxane est ajoutée une solution de soude 1N (20 mmoles). Le mélange est placé sous hydrogène pendant une nuit à pression et température ambiantes en présence de Pd/C à 10 % (42 mg). Après filtration du catalyseur, le solvant est évaporé, le résidu est repris par de l'eau puis acidifié par KHSO₄. Le précipité est filtré puis séché sous vide au dessicateur en présence de P₂O₅. On obtient le produit attendu sous la forme d'un solide blanc.

### Stade I : (6S)-N-[(6-Amino-2-méthyl-3 pyridyl)-méthyl]-4-oxo-3-(2-phénéthylamino)-4,6,7,8-tétrahydropyrrolo[1,2-a]pyrazine-6-carboxamide, dichlorhydrate

A 10 mmoles du composé obtenu dans le stade précédent et 11 mmoles du composé décrit dans la préparation A en solution dans le diméthylformamide sont ajoutées 11 mmoles de tétrafluoroborate de O-(1*H*-benzotriazol-1-yl)-N,N,N'N'-tétraméthyluronium et Il mmoles de diisopropyléthylamine. Après une nuit d'agitation à température ambiante, le solvant est évaporé. Le résidu obtenu est repris par de l'acétate d'éthyle. La phase organique est lavée, séchée puis évaporée. Le résidu obtenu est mis en solution dans l'acétate d'éthyle puis on fait passer à 0°C un courant d'acide chlorhydrique gaz pendant 30 minutes. Après une nuit d'agitation à température ambiante, le précipité formé est filtré, rincé à l'acétate d'éthyle et séché sous vide au dessicateur.

| *Microanalyse élémentaire:* | | | | |
|---|---|---|---|---|
| | *%C* | *%H* | *%N* | *%Cl* |
| *Calculé* | *56,22* | *5,74* | *17,10* | *14,43* |
| *Trouvé* | *56,93* | *5,73* | *17,34* | *14,60* |

### EXEMPLE 2 : (6R)-N-[(6-Amino-2-méthyl-3-pyridyl)-méthyl]-4-oxo-3-(2-phénéthylamino)-4,6,7,8-tétrahydropyrrolo[1,2-a]pyrazine-6-carboxamide, dichlorhydrate

Le produit attendu est obtenu à partir de (2*R*)-5-oxoprolinate de benzyle selon le procédé décrit dans l'exemple 1.

### EXEMPLE 3 : N-[(6-Amino-2-méthyl-3-pyridyl)-méthyl]-4-oxo-3-(2-phénéthylamino)-4,6,7,8-tétrahydropyrrolo[1,2-a]pyrazine-6-carboxamide, dichlorhydrate

Le produit attendu est obtenu à partir de (±)-5- oxoprolinate de benzyle selon le procédé décrit dans l'exemple 1.

### EXEMPLE 4: 1 (6S)-N-[(6-Amino-2-méthyl-3-pyridyl)-méthyl]-4-oxo-3-(2-phénéthylamino)-6,7,8,9-tétrahydro-4H-pyrido[1,2-a]pyrazine-6-carboxamide, dichlorhydrate

Le produit attendu est obtenu selon le procédé décrit dans l'exemple 1 à partir de (2*S*)-6-oxo-2-pipéridine carboxylate de benzyle.

### EXEMPLE 5: (3S)-N-[(6-Amino-2-méthyl-3-pyridyl)-méthyl]-5-oxo-6-(2-phénéthylamino)-2,3-dihydro-5H-[1,3]-thiazolo[3,2-a]pyrazine-3-carboxamide, dichlorhydrate

Le produit attendu est obtenu selon le procédé décrit dans l'exemple 1 à partir de (4*R*)-2-oxo-1,3-thiazolidine-4-carboxylate de benzyle.

### EXEMPLE 6 : 1 (6S)-N-[(6-Amino-2-méthyl-3-pyridyl)-méthyl]-3-[(2,2-diphényléthyl) amino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-diphényléthylamine et du composé décrit dans la préparation A.

| *Microanalyse élémentaire:* | | | | |
|---|---|---|---|---|
| | *%C* | *%H* | *%N* | *%Cl* |
| *Calculé* | *61,38* | *5,68* | *14,81* | *12,49* |
| *Trouvé* | *61,97* | *5,76* | *15,10* | *12,41* |

### EXEMPLE 7 : (6S)-N-[(6-Amino-2-méthyl-3-pyridyl)-méthyl]-3-[(3,4-diméthoxyphénéthyl)amino]-4-oxo-4,6,7,8-tétrahydropyrrolo[1,2-a]pyrazine-6-carboxamide, dichlorhydrate, monohydrate

Le produit attendu est obtenu selon le procédé décrit dans les stades F à I de l'exemple 1, à partir du composé décrit dans le stade E de l'exemple 1, de 3,4-diméthoxyphénéthylamine et du composé décrit dans la préparation A.

| *Microanalyse élémentaire:* | | | | |
|---|---|---|---|---|
| | *%C* | *%H* | *%N* | *%Cl* |
| *Calculé* | *52,73* | *6,02* | *14,76* | *12,45* |
| *Trouvé* | *53,39* | *6,26* | *14,70* | *13,00* |

### EXEMPLE 8 : (6S)-N-[(6-Amino-2-méthyl-3-pyridyl)-méthyl]-4-oxo-3-[(2-(4-pyridyl)éthyl)-amino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 4-(2-aminoéthyl)-pyridine et du composé décrit dans la préparation A.

### EXEMPLE 9 : (6S)-N-[(6-Amino-2-méthyl-3-pyridyl)-méthyl]-3-[(2-cyclohexyléthyl)-amino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2-cyclohexyléthylamine et du composé décrit dans la préparation A.

### EXEMPLE 10 : (2R)-N-[(6S)-6-{5[((6-Amino-2-méthyl-3-pyridyl)-méthyl)-amino]-carbonyl}-4-oxo-4,6,7,8-tétrahydropyrrolo[1,2-a]pyrazin-3-yl]-phénylalanine, chlorhydrate

Le produit attendu est obtenu selon le procédé décrit dans les stades F à I de l'exemple 1, à partir du composé décrit dans le stade E de l'exemple 1, de l'ester tert-butyliqne de la (2*R*)-phénylalanine et du composé décrit dans la préparation A.

### EXEMPLE 11 : (3S)-N-[(6-Amino-2-méthyl-3-pyridyl)-méthyl]-5-oxo-6-(2-phénéthyl amino)-2,3-dihydro-5H-[1,3]oxazolo[3,2-a]pyrazine-3-carboxamide, dichlorhydrate

Le produit attendu est obtenu selon le procédé décrit dans l'exemple 1 à partir de (4*R*)-2-oxo-1,3-oxazolidine-4-carboxylate de benzyle, dont la préparation est décrite dans Tet. Lett. 1995, 36 (37), 6595-6598.

### EXEMPLE 12: (6S)-N-[(6-Amino-2-méthyl-3-pyridyl)-méthyl]-3-benzylamino-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de benzylamine et du composé décrit dans la préparation A.

| *Microanalyse élémentaire :* | | | | |
|---|---|---|---|---|
| | *%C* | *% H* | *%N* | *%Cl* |
| *Calculé* | *55,35* | *5,49* | *17,60* | *14,85* |
| *Trouvé* | *55.97* | *5.33* | *17,62* | *14,47* |

### EXEMPLE 13 : (6S)-N-[(6-Amino-2-méthyl-3-pyridyl)-méthyl]-4-oxo-3-(3-phénylpropylamino)-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 3-phénylpropylamine et du composé décrit dans la préparation A.

| *Microanalyse élémentaire*: | | | | |
|---|---|---|---|---|
| | *%C* | *%H* | *%N* | *%CI* |
| *Calculé* | *57,03* | *5,98* | *16,63* | *14, 03* |
| *Trouvé* | *57,23* | *5,94* | *16,45* | *14,32* |

### EXEMPLE 14 : (6S)-N-[(6-Amino-2-méthyl-3-pyridyl)-méthyl]-3-diphénylméthylamino-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de diphénylméthylamine et du composé décrit dans la préparation A.

| *Microanalyse élémentaire:* | | | | |
|---|---|---|---|---|
| | *%C* | *%H* | *%N* | *%Cl* |
| *Calculé* | *60,76* | *5,46* | *15,18* | *12.81* |
| *Trouvé* | *60,59* | *5,53* | *15,11* | *13,62* |

### EXEMPLE 15 : (6S)-N-[(6-Amino-2-méthyl-3-pyridyl)-méthyl]-4-oxo-3-[2-(2-pyridyl) - éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 2-(2-pyridyl)-éthylamine et du composé décrit dans la préparation A.

| *Microanalyse élémentaire :* | | | | |
|---|---|---|---|---|
| | *%C* | *%H* | *%N* | *%Cl* |
| *Calculé* | *49,96* | *5,34* | *18,54* | *20,11* |
| *Trouvé* | *50,27* | *5,23* | *18,46* | *20.08* |

### EXEMPLE 16 : (6S)-N-[(6-Amino-2-méthyl-3-pyridyl)-méthyl]-4-oxo-3-[2-(3-pyridyl) -éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 2-(3-pyridyl)-éthylamine et du composé décrit dans la préparation A.

| *Microanalyse élémentaire* | | | | |
|---|---|---|---|---|
| | *%C* | *%H* | *%N* | *%Cl* |
| *Calculé* | *49, 96* | *5, 34* | *18,54* | *20,11* |
| *Trouvé* | *49,13* | *5,32* | *18,08* | *20,00* |

### EXEMPLE 17 : (6S)-N-[(6-Amino-2-méthyl-3-pyridyl)-méthyl]-3-(indan-2-yl-méthylamino)-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, d'indan-2-yl-méthylamine et du composé décrit dans la préparation A.

| *Microanalyse élémentaire:* | | | | |
|---|---|---|---|---|
| | *%C* | *%H* | *%N* | *%Cl* |
| *Calculé* | *58,03* | *5,84* | *16,24* | *13,70* |
| *Trouvé* | *57,90* | *5,69* | *16,20* | *13,05* |

### EXEMPLE 18 : (6S)-N-[(6-Amino-2-méthyl-3-pyridyl)-méthyl]-3-[(1,1'-biphényl)-4-yl-méthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (1,1'-biphényl )-4-yl-méthylamine et du composé décrit dans la préparation A.

### EXEMPLE 19 : (6S)-N-[(6-Amino-2-méthyl-3-pyridyl)-méthyl]-3-(2-méthyl-2-phénylpropylamino)-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2-méthyl-2-phenylpropylamine et du composé décrit dans la préparation A.

| *Microanalyse élémentaire* | | | | |
|---|---|---|---|---|
| | *%C* | *%H* | *%N* | *%Cl* |
| *Calculé* | *57,80* | *6,21* | *16.18* | *13.65* |
| *Trouvé* | *57,29* | *6,49* | *15.93* | *14.22* |

### EXEMPLE 20 : (6S)-N-[(6-Amino-2-méthyl-3-pyridyl)-méthyl]-4-oxo-3-[(2 α)-2-(2-pyridyl)-2-phénéthylamino]-4,6,7,8-tétrahydropyrrolo[1,2-a] pyrazine-6-carboxamide, trichlorhydrate

### Stade A : (2α)-2-(2-Pyridyl)-2-phénéthylamine

Le produit attendu est obtenu par dédoublement de la (±)-2-(2-pyridyl)-2-phénéthylamine (décrite dans J. Am. Chem. Soc. 1971, 93, 5542) à l'aide d'acide D-dibenzoyl-tartriqué.

### Stade B : (6S)-N-[(6-Amino-2-méthyl-3-pyridyl)-méthyl]-4-oxo-3-[(2α)-2-(2-pyridyl)-2-phénéthylamino]-4,6,7,8-tétrahydropyrrolo[1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, du composé obtenu dans le stade précédent et du composé décrit dans la préparation A.

| *Microanalyse élémentaire* | | | | |
|---|---|---|---|---|
| | *%C* | *% H* | *%N* | *%Cl* |
| *Calculé* | *55,59* | *5.33* | *16,21* | *17,58* |
| *Trouvé* | *54,83* | *5,46* | *15, 83* | *18,41* |

### EXEMPLE 21 : (6S)-N-[(6-Amino-2-méthyl-3-pyridyl)-méthyl]-4-oxo-3-[(2β)-2-(2-pyridyl)-2-phénéthylamino]-4,6,7,8-tétrahydropyrrolo[1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, de (2β)-2-(2-pyridyl)-2-phénéthylamine et du composé décrit dans la préparation A.

### EXEMPLE 22 : (6S)-N-[(6-Amino-2-méthyl-3-pyridyl)-méthyl]-3-[2,2-di-(2-pyridyl)-éthylamino]-4-oxo-4,6,7,8-tétrahydropyrrolo[1,2-a]pyrazine-6-carboxamide, tétrachlorhydrate

Le produit attendu est obtenu selon le procédé décrit dans les stades F à I de l'exemple 1, à partir du composé décrit dans le stade E de l'exemple 1, de 2,2-di-(2-pyridyl)-éthylamine et du composé décrit dans la préparation A.

| *Microanalyse élémentaire :* | | | | |
|---|---|---|---|---|
| | *%C* | *%H* | *%N* | *%Cl* |
| *Calculé* | *50,48* | *5,02* | *17,44* | *22,07* |
| *Trouvé* | *50,73* | *5,18* | *17,28* | *22,56* |

### EXEMPLE 23 : (6S)-N-[(6-Amino-2-méthyl-3-pyridyl)-méthyl]-3-[2,2-bis-(4-méthoxy-phényl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(4-méthoxyphényl)-éthylamine et du composé décrit dans la préparation A.

### EXEMPLE 24 : (6S)-N-[(6-Amino-2-méthyl-3-pyridyl)-méthyl]-3-[2,2-bis-(4-hydroxyphényl)-éthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(4-hydroxyphényl)-éthylamine et du composé décrit dans la préparation A.

### EXEMPLE 25 : (6S)-N-[(6-Amino-2-méthyl-3-pyridyl)-méthyl]-3-[2,2-bis-(4-chlorophényl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(4-chlorophényl)-éthylamine et du composé décrit dans la préparation A.

### EXEMPLE 26 : (6S)-N-[(6-Amino-2-méthyl-3-pyridyl)-méthyl]-3-[2,2-di-(p-tolyl)-éthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1; de 2,2-di-(p-tolyl)-éthylamine et du composé décrit dans la préparation A.

### EXEMPLE 27 : (6S)-N-[(6-Amino-2-méthyl-3-pyridyl)-méthyl]-3-[2,2-bis-(4-fluorophényl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(4-fluorophényl)-éthylamine et du composé décrit dans la préparation A.

### EXEMPLE 28 : (6S)-N-[(6-Amino-2-méthyl-3-pyridyl)-méthyl]-3-(2,2-dicyclohexyléthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-dicyclohexyl-éthylamine et du composé décrit dans la préparation A.

| *Microanalyse élémentaire:* | | | | |
|---|---|---|---|---|
| | *%C* | *%H* | *%N* | *%Cl* |
| *Calculé* | *60,10* | *7,65* | *14,39* | *12,23* |
| *Trouvé* | *60.27* | *7,67* | *14,50* | *12,29* |

### EXEMPLE 29 : (6S)-N-[(6-Amino-2-méthyl-3-pyridyl)-méthyl]-3-[2,2-bis-(2,4-difluorophényl)-éthylamino]-4-oxo-4,6,7,8-tétrahydropyrrolo[ 1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(2,4-difluorophényl)-éthylamine et du composé décrit dans la préparation A.

### EXEMPLE 30 : (6S)-N-[(6-Amino-2-méthyl-3-pyridyl)-méthyl]-3-(2,2-bis-(2,6-diméthylphényl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(2,6-diméthylphényl)-éthylamine et du composé décrit dans la préparation A.

### EXEMPLE 31 : (6S)-N-[(6-Amino-2-méthyl-3-pyridyl)-méthyl]-3-[2,2-bis-(6-éthyl-2-pyridyl)-éthylamino]-4-oxo-4,6,7,8-tétrahydropyrrolo[1,2-a]pyrazine-6-carboxamide, tétrachlorhydrate

Le produit attendu est obtenu selon le procédé décrit dans les stades F à I de l'exemple 1, à partir du composé décrit dans le stade E de l'exemple 1, de 2,2-bis-(6-éthyl-2-pyridyl)-éthylamine et du composé décrit dans la préparation A.

### EXEMPLE 32 : (6S)-N-[(6-Amino-2-méthyl-3-pyridyl)-méthyl]-4-oxo-3-[(2R)-2-(1-naphtyl)-2-(2-pyridyl)-éthylamino]-4,6,7,8-tétrahydropyrrolo[1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(1-naphtyl)-2-(2-pyridyl)-éthylamine et du composé décrit dans la préparation A.

### EXEMPLE 33 : (6S)-N-[(6-Amino-2-méthyl-3-pyridyl)-méthyl]-4-oxo-3-[(2S)-2-(1-naphtyl)-2-(2-pyridyl)-éthylamino]-4,6,7,8-tétrahydropyrrolo[ 1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(1-naphtyl)-2-(2-pyridyl)-éthylamine et du composé décrit dans la préparation A.

### EXEMPLE 34 : (6S)-N-[(6-Amino-2-méthyl-3-pyridyl)-méthyl]-4-oxo-3-[(2R)-2-(2-naphtyl)-2-(2-pyridyl)-éthylamino]-4,6,7,8-tétrahydropyrrolo[1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(2-naphtyl)-2-(2-pyridyl)-éthylamine et du composé décrit dans la préparation A.

### EXEMPLE 35 : (6S)-N-[(6-Amino-2-méthyl-3-pyridyl)-méthyl]-4-oxo-3-[(2S)-2-(2-naphtyl)-2-(2-pyridyl)-éthylamino]-4,6,7,8-tétrahydropyrrolo[1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(2-naphtyl)-2-(2-pyridyl)-éthylamine et du composé décrit dans la préparation A.

### EXEMPLE 36 : (6S)-N-[(6-Amino-2-méthyl-3-pyridyl)-méthyl]-4-oxo-3-[(2R)-2-(2-pyridyl)-2-phénylbutylamino]-4,6,7,8-tétrahydropyrrolo[1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(2-pyridyl)-2-phénylbutylamine et du composé décrit dans la préparation A.

### EXEMPLE 37 : (6S)-N-[(6-Amino-2-methyl-3-pyridyl)-méthyl]-4-oxo-3-[(2S)-2-(2-pyridyl)-2-phénylbutylamino]-4,6,7,8-tétrahydropyrrolo[1,2-a] pyrazine-6-carboxamide, trichlorhydrate

Le produit attendu est obtenu selon le procédé décrit dans les stades F à 1 de l'exemple 1, à partir du composé décrit dans le stade E de l'exemple 1, de (2*S*)-2-(2-pyridyl)-2-phénylbutylamine et du composé décrit dans la préparation A.

### EXEMPLE 38 : (6S)-N-[(6-Amino-2-méthyl-3-pyridyl)-méthyl]-3-[(9H-fluorén-9-yl)-méthylamino]-4-oxo-4,6,7,8-tétrahydropyrrolo[1,2-a]pyrazine-6-carboxamide, dichlorhydrate

Le produit attendu est obtenu selon le procédé décrit dans les stades F à 1 de l'exemple 1, à partir du composé décrit dans le stade E de l'exemple 1. de (9H-fluorén-9-yl)-méthylamine et du composé décrit dans la préparation A.

### EXEMPLE 39 : (6S)-N-[(6-Amino-2-méthyl-3-pyridyl)-méthyl]-3-[(9,10-dihydro-9-anthryl)-méthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de (9,10-dihydro-9-anthryl)-méthylamine et du composé décrit dans la préparation A.

### EXEMPLE 40 : (6S)-N-[(6-Amino-2-méthyl-3-pyridyl)-méthyl]-3-[(10,11-dihydro-5H-dibenzo[a,d]cyclohepten-5-yl)-méthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (10,11-dihydro-5H-dibenzo[a,d]cyclohepten-5-yl)-méthylamine et du composé décrit dans la préparation A.

| *Microanalyse élémentaire:* | | | | |
|---|---|---|---|---|
| | *%C* | *%H* | *%N* | *%Cl* |
| *Calculé* | *62.73* | *5,77* | *14,16* | *11,95* |
| *Trouvé* | *63,02* | *5,83* | *14,09* | *11,49* |

### EXEMPLE 41 : (6S)-N-[(6-Amino-2-méthyl-3-pyridyl)-méthyl]-4-oxo-3-[(9H-xanthén-9-yl)-méthylamino]4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (9H-xanthén-9-yl)-méthylamine et du composé décrit dans la préparation A.

### EXEMPLE 42 : (6S)-N-[(6-Amino-2-méthyl-3-pyridyl)-méthyl]-4-oxo-3-[(9H-thioxanthén-9-yl)-méthylamino]-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de (9*H*-thioxanthén-9-yl)-méthylamine et du composé décrit dans la préparation A.

### EXEMPLE 43 : (6S)-N-[(6-Amino-2-méthyl-3-pyridyl)-méthyl-3-[(10-méthyl-9,10-dihydro-9-acridinyl)-méthylamino]-4-oxo-4,6,7,8-tétrahydro-pyrrolo [1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (10-méthyl-9.10-dihydro-9-acridinyl)-méthylamine et du composé décrit dans la préparation A.

### EXEMPLE 44 : (6S)-N-[(6-Amino-2-méthyl-3-pyridyl)-méthyl]-3-[(9H-indéno [2,1-b]pyridin-9-yl)-méthylamino]-4-oxo-4,6,7,8-tétrahydro-pyrrolo [1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (9*H*-indéno [2,1-b]pyridin-9-yl)-méthylamine et du composé décrit dans la préparation A.

### EXEMPLE 45 : (6S)-N-[(6-Amino-2-méthyl-3-pyridyl)-méthyl]-3-[(5,10-dihydrobenzo[g]quinolin-10-yl)-méthylamino]-4-oxo-4,6,7,8-tétrahydropyrrolo[1,2-a]pyrazine-6-carboxamide, trichlorhydrate

Le produit attendu est obtenu selon le procédé décrit dans les stades F à I de l'exemple 1. à partir du composé décrit dans le stade E de l'exemple 1, de (5,10-dihydrobenzo[g]quinolin-10-yl)-méthylamine et du composé décrit dans la préparation A.

### EXEMPLE 46 : (6S)-N-[(6-Amino-2-méthyl-3-pyridyl)-méthyl]-3-[(6,11-dihydro-5H-benzo[5,6]cyclohepta[1,2-b]pyridin-11-yl)-méthylamino]-4-oxo-4,6,7, 8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (6,11-dihydro-5*H*-benzo[5,6]cyclohepta[1,2-b]pyridin-11-yl)-méthylamine et du composé décrit dans la préparation A.

### EXEMPLE 47 : (6S)-N-[(6-Amino-2-méthyl-3-pyridyl)-méthyl]-3-[(10,11-dihydro-4,6-diaza-5H-dibenzo[a,d]cyclohepten-5-yl)-méthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (10,11-dihydro-4,6-diaza-5*H*-dibenzo[a,d]cyclohepten-5-yl)-méthylamine et du composé décrit dans la préparation A.

### EXEMPLE 48 : (6S)-N-[(6-Amino-2-méthyl-3-pyridyl)-méthyl]-4-oxo-3-[2-(5-phényl-2-pyridyl)-éthylamino]-4,6,7,8-tétrahydropyrrolo[1,2-a]pyrazine-6-carboxamide, dichlorhydrate

Le produit attendu est obtenu selon le procédé décrit dans les stades F à 1 de l'exemple 1, à partir du composé décrit dans le stade E de l'exemple 1, de 2-(5-phényl-2-pyridyl)-éthylamine et du composé décrit dans la préparation A.

### EXEMPLE 49 : (6S)-N-[(6-Amino-2-méthyl-3-pyridyl)-méthyl]-4-oxo-3-[2-(5-butyl-2-pyridyl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 2-(5-butyl-2-pyridyl)-éthylamine et du composé décrit dans la préparation A.

### EXEMPLE 50 : (6S)-N-[(6-Amino-2-méthyl-3-pyridyl)-méthyl]-3-[2-(1-naphtyl)-éthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2-(1-naphtyl)-éthylamine et du composé décrit dans la préparation A.

| *Microanalyse élémentaire :* | | | | |
|---|---|---|---|---|
| | *%C* | *%H* | *%N* | *%Cl* |
| *Calculé* | *59,89* | *5,58* | *15,52* | *13,09* |
| *Trouvé* | *60,47* | *5,84* | *15,57* | *12,76* |

### EXEMPLE 51 : (6S)-N-[(6-Amino-2-méthyl-3-pyridyl)-méthyl]-3-[2-(2-naphtyl)-éthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2-(2-naphtyl)-éthylamine et du composé décrit dans la préparation A.

| *Microanalyse élémentaire* : | | | | |
|---|---|---|---|---|
| | *%C* | *%H* | *%N* | *%Cl* |
| *Calculé* | *59,89* | *5,58* | *15,52* | *13,09* |
| *Trouvé* | *60,66* | *5,71* | *15,65* | *12,58* |

### EXEMPLE 52 : (6S)-N-[(6-Amino-2-méthyl-3-pyridyl)-méthyl]-4-oxo-3-[2-(5-éthoxy-6-méthyl-2-pyridyl)-éthylamino]-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2-(5-éthoxy-6-méthyl-2-pyridyl)-éthylamine et du composé décrit dans la préparation A.

| Micronalyse *élémentaire* : | | | | |
|---|---|---|---|---|
| | *%C* | *%H* | *%N* | *%Cl* |
| *Calculé* | *54,55* | *6,04* | *17,81* | *12.88* |
| *Trouvé* | *55,70* | *6,13* | *17,71* | *13,18* |

### EXEMPLE 53 : (6S)-N-[(6-Amino-2-méthyl-3-pyridyl)-méthyl]-4-oxo-3-[2-(6,7-dihydro-5H-cyclopenta[b]pyridin-2-yl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 2-(6,7-dihydro-5*H*-cyclopenta[b]pyridin-2-yl)-éthylamine et du composé décrit dans la préparation A.

### EXEMPLE 54 : (6S)-N-[(6-Amino-2-méthyl-3-pyridyl)-méthyl]-3-[1,3-di-(2-pyridyl)-2-propanamino]-4-oxo-4,6,7,8-tétrahydropyrrolo[1,2-a]pyrazine-6-carboxamide, tétrachlorhydrate

Le produit attendu est obtenu selon le procédé décrit dans les stades F à I de l'exemple 1, à à partir du composé décrit dans le stade E de l'exemple 1, de 1,3-di-(2-pyridyl)-2-propanamine et du composé décrit dans la préparation A.

### EXEMPLE 55 : (6S)-N-[(6-Amino-2-méthyl-3-pyridyl)-méthyl]-3-[1,3-diphényl-2-propanamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 1,3-diphényl-2-propanamine et du composé décrit dans la préparation A.

### EXEMPLE 56: (6S)-N-[(6-Amino-2-méthyl-3-pyridyl)-méthyl]-3-[(2α)-3,3,3-trifluoro-2-phényl-1-propanamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (2α)-3,3,3-trifluoro-2-phényl-1 -propanamine et du composé décrit dans la préparation A.

### EXEMPLE 57 : (6S)-N-[(6-Amino-2-méthyl-3-pyridyl)-méthyl]-3-[(2β)-3,3,3-trifluoro-2-phényl-1-propanamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (2β)-3,3,3-trifluoro-2-phényl-1-propanamine et du composé décrit dans la préparation A.

### EXEMPLE 58 : (6S)-N-[(6-Amino-2-méthyl-3-pyridyl)-méthyl]-3-[(2R)-3,3,3-trifluoro-2-cyclohexyl-1 -propanamino]-4-oxo-4,6,7,8-tétrahydropyrrolo[1,2-a]pyrazine-6-carboxamide, dichlorhydrate

Le produit attendu est obtenu selon le procédé décrit dans les stades F à 1 de l'exemple 1, à partir du composé décrit dans le stade E de l'exemple 1, de (2*R*)-3,3,3-trifluoro-2-cyclohexyl-1-propanamine et du composé décrit dans la préparation A.

### EXEMPLE 59 : (6S)-N-[(6-Amino-2-méthyl-3-pyridyl)-méthyl]-3-[(2S)-3,3,3-trifluoro-2-cyclohexyl-1-propanamino]-4-oxo-4,6,7,8-tétrahydropyrrolo[1,2-a]pyrazine-6-carboxamide, dichlorhydrate

Le produit attendu est obtenu selon le procédé décrit dans les stades F à 1 de l'exemple 1, à partir du composé décrit dans le stade E de l'exemple 1, de (2*S*)-3,3,3-trifluoro-2-cyclohexyl-1-propanamine et du composé décrit dans la préparation A.

### EXEMPLE 60 : (6S)-N-[(6-Amino-2-méthyl-3-pyridyl)-méthyl]-3-(2-indanamino)-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2-indanamine et du composé décrit dans la préparation A.

| *Microanalyse élémentaire :* | | | | |
|---|---|---|---|---|
| | *%C* | *%H* | *%N* | *%Cl* |
| *Calculé* | *57,26* | *5,61* | *16,69* | *14,08* |
| *Trouvé* | *57,75* | *5,54* | *16,72* | *14,07* |

### EXEMPLE 61 : (6S)-N-[(6-Amino-2-méthyl-3-pyridyl)-méthyl]-3-(1-phényl-3-azétidinamino)-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 1-phényl-3-azétidinamine et du composé décrit dans la préparation A.

### EXEMPLE 62 : (6S)-N-[(6-Amino-2-méthyl-3-pyridyl)-méthyl]-3-(1-benzhydryl-3-azétidinamino)-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1. à partir du composé décrit dans le stade E de l'exemple 1, de 1-benzhydryl-3-azétidinamine et du composé décrit dans la préparation A.

### EXEMPLE 63 : (6S)-N-[(6-Amino-2-méthyl-3-pyridyl)-méthyl]-3-(1-(2-naphtyl)-3-azétidinamino)-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 1-(2-naphtyl)-3-azétidinamine et du composé décrit dans la préparation A.

### EXEMPLE 64 : (6S)-N-[(6-Amino-2-méthyl-3-pyridyl)-méthyl]-3-(4,4-diphényl-cyclohexylamino)-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 4,4-diphénylcyclohexylamine et du composé décrit dans la préparation A.

### EXEMPLE 65 : (6S)-N-[(6-Amino-2,5-diméthyl-3-pyridyl)-méthyl]-3-(2,2-diphényléthylamino)-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-diphényl-éthylamine et du composé décrit dans la préparation B.

### EXEMPLE 66 : (6S)-N-[(6-Amino-2,4-diméthyl-3-pyridyl)-méthyl]-3-(2,2-diphényléthylamino)4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2.2-diphényl-éthylamine et du composé décrit dans la préparation C.

### EXEMPLE 67 : (6S)-N-[(6-Amino-2-éthyl-3-pyridyl)-méthyl]-3-(2,2-diphényléthylamino)-4-oxo-4,6,7,8-tétrahydropyrrolo[1,2-a]pyrazine-6-carboxamide, dichlorhydrate

Le produit attendu est obtenu selon le procédé décrit dans les stades F à 1 de l'exemple 1, à partir du composé décrit dans le stade E de l'exemple 1, de 2,2-diphényl-éthylamine et du composé décrit dans la préparation D.

### EXEMPLE 68 : (6S)-N-[(6-Amino-2,5-diméthyl-3-pyridyl)-méthyl]-3-[2-(2-pyridyl)-éthylamino]-4-oxo-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 2-(2-pyridyl)-éthylamine et du composé décrit dans la préparation B.

| *Microanalyse élémentaire :* | | | | |
|---|---|---|---|---|
| | *%C* | *%H* | *%N* | *%Cl* |
| *Calculé* | *49,88* | *5,53* | *17,71* | *21,12* |
| *Trouvé* | *50,07* | *5,65* | *17,68* | *20,99* |

### EXEMPLE 69 : (6S)-N-[(6-Amino-2,4-diméthyl-3-pyridyl)-méthyl]-3-[2-(2-pyridyl)-éthylamino]-4-oxo-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 2-(2-pyridyl)-éthylamine et du composé décrit dans la préparation C.

### EXEMPLE 70 : (6S)-N-[(6-Amino-2-éthyl-3-pyridyl)-méthyl]-3-[2-(2-pyridyl)-éthylamino]4-oxo-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de 2-(2-pyridyl)-éthylamine et du composé décrit dans la préparation D.

### EXEMPLE 71 : (6S)-N-[(2-Ethylcarbamoylméthoxy)-benzyl]-4-oxo-3-(2-phénéthylamino)-4,6,7,8-tétrahydropyrrolo[1,2-a]pyrazine-6-carboxamide

Le produit attendu est obtenu selon le procédé décrit dans les stades F à I de l'exemple 1, à partir du composé décrit dans le stade E de l'exemple 1, de 2-phénéthylamine et du composé décrit dans la préparation E.

| *Microanalyse élémentaire :* | | | |
|---|---|---|---|
| | *%C* | *%H* | *%N* |
| *Calculé* | *66,24* | *6,38* | *14,30* |
| *Trouvé* | *65,52* | *6,33* | *14,18* |

### EXEMPLE 72 : (6S)-N-[3-(1H-imidazol-4-yl)-propyl]-4-oxo-3-(2,2-diphényléthylamino)-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-diphényl-éthylamine et de 3-(1*H*-imidazol-4-yl)-propylamine décrite dans Liebigs Ann. Chem.. 1992, 317-323.

| *Microanalyse élémentaire :* | | | | |
|---|---|---|---|---|
| | *%C* | *%H* | *%N* | *%Cl* |
| *Calculé* | *61,34* | *5,86* | *15,33* | *11,64* |
| *Trouvé* | *61,43* | *5,83* | *14,91* | *11,97* |

### EXEMPLE 73 : (6S)-N-[(6-Amino-2-méthyl-3-pyridyl)-méthyl]-3-[2,2-bis-(2-fluoro-4-méthoxyphényl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à 1 de l'exemple 1, à partir du composé décrit dans le stade E de l'exemple 1, de 2,2-bis-(2-fluoro-4-méthoxyphényl)-éthylamine et du composé décrit dans la préparation A.

### EXEMPLE 74 : (6S)-N-[(6-Amino-2-méthyl-3-pyridyl)-méthyl]-3-[2,2-bis-(4-fluoro-2-méthoxyphényl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(4-fluoro-2-méthoxyphényl)-éthylamine et du composé décrit dans la préparation A.

### EXEMPLE 75 : (6S)-N-[(6-Amino-2-méthyl-3-pyridyl)-méthyl]-3-[2,2-bis-(2-fluoro-p-tolyl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(2-fluoro-p-tolyl)-éthylamine et du composé décrit dans la préparation A.

### EXEMPLE 76 : (6S)-N-[(6-Amino-2-méthyl-3-pyridyl)-méthyl]-4-oxo-3-[(2R)-2-(5-méthyl-2-pyridyl)-2-(4-méthoxyphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(5-méthyl-2-pyridyl)-2-(4-méthoxyphényl)-éthylamine et du composé décrit dans la préparation A.

### EXEMPLE 77 : (6S)-N-[(6-Amino-2-méthyl-3-pyridyl)-méthyl]-4-oxo-3-[(2S)-2-(5-méthyl-2-pyridyl)-2-(4-méthoxyphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(5-méthyl-2-pyridyl)-2-(4-méthoxyphényl)-éthylamine et du composé décrit dans la préparation A.

### EXEMPLE 78 : (6S)-N-[(6-Amino-2-méthyl-3-pyridyl)-méthyl]-4-oxo-3-[(2R)-2-(2-pyridyl)-2-(3,4-diméthylphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(2-pyridyl)-2-(3,4-diméthylphényl)-éthylamine et du composé décrit dans la préparation A.

### EXEMPLE 79 : (6S)-N-[(6-Amino-2-méthyl-3-pyridyl)-méthyl]-4-oxo-3-[(2S)-2-(2-pyridyl)-2-(3,4-diméthylphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(2-pyridyl)-2-(3,4-diméthylphényl)-éthylamine et du composé décrit dans la préparation A.

### EXEMPLE 80 : (6S)-N-[(6-Amino-2-méthyl-3-pyridyl)-méthyl]-4-oxo-3-[(2R)-2-(2-pyridyl)-2-(2,4-diméthylphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(2-pyridyl)-2-(2,4-diméthylphényl)-éthylamine et du composé décrit dans la préparation A.

### EXEMPLE 81 : (6S)-N-[(6-Amino-2-méthyl-3-pyridyl)-méthyl]-4-oxo-3-[(2S)-2-(2-pyridyl)-2-(2,4-diméthylphényl)-éthylamino]-4,6,7,8-tétrahydropyrrolo[1,2-a] pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(2-pyridyl)-2-(2,4-diméthylphényl)-éthylamine et du composé décrit dans la préparation A.

### EXEMPLE 82 : (6S)-N-[(6-Amino-2-méthyl-3-pyridyl)-méthyl]-4-oxo-3-[(2R)-2-(2-pyridyl)-2-(4-éthylphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(2-pyridyl)-2-(4-éthylphényl)-éthylamine et du composé décrit dans la préparation A.

### EXEMPLE 83 : (6S)-N-[(6-Amino-2-méthyl-3-pyridyl)-méthyl]-4-oxo-3-[(2S)-2-(2-pyridyl)-2-(4-éthylphényl)-éthylaminol-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(2-pyridyl)-2-(4-éthylphényl)-éthylamine et du composé décrit dans la préparation A.

### EXEMPLE 84 : (6S)-N-[(6-Amino-2-méthyl-3-pyridyl)-méthyl]-3-[2,2-bis-(2-fluoro-4-hydroxyphényl)-éthylamino]-4-oxo-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(2-fluoro-4-hydroxyphényl)-éthylamine et du composé décrit dans la préparation A.

### EXEMPLE 85 : (6S)-N-[(6-Amino-2-méthyl-3-pyridyl)-méthyl]-3-[2,2-bis-(2,4-diméthoxyphényl)-éthylamino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-bis-(2,4-diméthoxyphényl)-éthylamine et du composé décrit dans la préparation A.

### EXEMPLE 86 : (6S)-N-[2-(Ethylcarbamoylméthoxy)-benzyl]-4-oxo-3-[2-(2-pyridyl)-éthylamino]-4,6,7,8-tétrahydropyrrolo[1,2-a]pyrazine-6-carboxamide, chlorhydrate

Le produit attendu est obtenu selon le procédé décrit dans les stades F à I de l'exemple 1, à partir du composé décrit dans le stade E de l'exemple 1, de 2-(2-pyridyl)-éthylamine et du composé décrit dans la préparation E.

### EXEMPLE 87 : (6S)-N-[5-Chloro-2-(éthylcarbamoylméthoxy)-benzyl]-4-oxo-3-[2-(2-pyridyl)-éthylamino]-4,6,7,8-tétrahydropyrrolo[1,2-a]pyrazine-6-carboxamide, chlorhydrate

Le produit attendu est obtenu selon le procédé décrit dans les stades F à I de l'exemple 1, à partir du composé décrit dans le stade E de l'exemple 1, de 2-(2-pyridyl)-éthylamine et du composé décrit dans la préparation F.

### EXEMPLE 88 : (6S)-N-(2,5-Dichlorobenzyl)-4-oxo-3-[2-(2-pyridyl)-éthylamino]-4,6,7,8-tétrahydropyrrolo[1,2-a]pyrazine-6-carboxamide, chlorhydrate

Le produit attendu est obtenu selon le procédé décrit dans les stades F à I de l'exemple 1, à partir du composé décrit dans le stade E de l'exemple 1, de 2-(2-pyridyl)-éthylamine et de 2,5-dichlorobenzylamine.

### EXEMPLE 89 : (6S,8R)-N-[(6-Amino-2-méthyl-3-pyridyl)-méthyl]-3-[(2,2-diphényléthyl)-amino]-8-hydroxy-4-oxo-4,6,7,8-tétrahydropyrrolo[1,2-a]pyrazine-6-carboxamide, dichlorhydrate

### Stade A : (2S,4R)-4-Hydroxy-5-oxo-2-pyrrolidine carboxylate de benzyle

Le produit attendu est obtenu selon le procédé décrit dans J. Org. Chem. 1996, 61 (14), 4838-41.

### Stade B : (6S, 8R)-N-[(6-Amino-2-méthyl-3-pyridyl)-méthyl]-3-[(2,2-diphényléthyl)-amino]-8-hydroxy-4-oxo-4,6,7,8-tétrahydropyrrolo[1,2-a]pyrazine-6-carboxamide, dichlorhydrate

Le produit attendu est obtenu selon le procédé décrit dans l'exemple 1, à partir du composé obtenu dans le stade précédent, de 2,2-diphényléthylamine et du composé décrit dans la préparation A.

### EXEMPLE 90 : (6S,8S)-N-[(6-Amino-2-méthyl-3-pyridyl)-méthyl]-3-[(2,2-diphényléthyl)-amino]-8-hydroxy-4-oxo-4,6,7,8-tétrahydropyrrolo[1,2-a]pyrazine-6-carboxamide, dichlorhydrate

### Stade A : (2S,4S)-4-Hydroxy-5-oxo-2-pyrrolidine carboxylate de benzyle

Le produit attendu est obtenu selon le procédé décrit dans J. Org. Chem. 1996, 61 (14), 4838-41.

### Stade B : (6S, 8S)-N-[(6-Amino-2-méthyl-3-pyridyl)-méthyl]-3-[(2,2-diphényléthyl)-amino]-8-hydroxy-4-oxo-4,6,7,8-tétrahydropyrrolo[1,2-a]pyrazine-6-carboxamide, dichlorhydrate

Le produit attendu est obtenu selon le procédé décrit dans l'exemple 1. partir du composé obtenu dans le stade précédent, de 2,2-diphényléthylamine et du composé décrit dans la préparation A.

### EXEMPLE 91 : (6S,8R)-N-[(6-Amino-2-méthyl-3-pyridyl)-méthyl]-3-[(2,2-diphényléthyl)-amino]-8-méthoxy-4-oxo-4,6,7,8-tétrahydropyrrolo[1,2-a]pyrazine-6-carboxamide, dichlorhydrate

### Stade A : (2S,4R)-4-Méthoxy-5-oxo-2-pyrrolidine carboxylate de benzyle

Le produit attendu est obtenu selon le procédé décrit dans Indian. J. Chem. (B) 1996, 35 (11), 1221-24 à partir du composé décrit dans le stade A de l'exemple 89.

### Stade B : (6S,8R)-N-[(6-Amino-2-méthyl-3-pyridyl)-méthyl]-3-[(2,2-diphényléthyl)-amino]-8-méthoxy-4-oxo-4,6,7,8-tétrahydropyrrolo[1.2-a]pyrazine-6-carboxamide, dichlorhydrate

Le produit attendu est obtenu selon le procédé décrit dans l'exemple 1, à partir du composé obtenu dans le stade précédent, de 2,2-diphényléthylamine et du composé décrit dans la préparation A.

### EXEMPLE 92 : (6S,8S)-N-[(6-Amino-2-méthyl-3-pyridyl)-méthyl]-3-[(2,2-diphényléthyl)-amino]-8-méthoxy-4-oxo-4,6,7,8-tétrahydropyrrolo[1,2-a]pyrazine-6-carboxamide, dichlorhydrate

### Stade A : (2S, 4S)-4-Méthoxy-5-oxo-2-pyrrolidine carboxylate de benzyle

Le produit attendu est obtenu selon le procédé décrit dans Indian. J. Chem. (B) 1996, 35 (11), 1221-24, à partir du composé décrit dans le stade A de l'exemple 90.

### Stade B : (6S, 8S)-N-[(6-Amino-2-méthyl-3-pyridyl)-méthyl]-3 -[(2,2-diphényléthyl)-amino]-8-méthoxy-4-oxo-4,6,7,8-tétrahydropyrrolo[1,2-a]pyrazine-6-carboxamide, dichlorhydrate

Le produit attendu est obtenu selon le procédé décrit dans l'exemple 1, à partir du composé obtenu dans le stade précédent, de 2,2-diphényléthylamine et du composé décrit dans la préparation A.

### EXEMPLE 93 : (6S)-N-[(6-Amino-2-hydroxyméthyl-3-pyridyl)-méthyl]-3-[(2,2-diphényléthyl)amino]-4-oxo-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de 2,2-diphényléthylamine et du composé décrit dans la préparation G.

### EXEMPLE 94 : (3R)-N-[(6-Amino-2-méthyl-3-pyridyl)-méthyl]-6-[(2,2-diphényléthyl) amino]-1-méthyl-5-oxo-1,2,3,5-tétrahydroimidazo[1,2-a]pyrazine-3-carboxamide, dichlorhydrate

### Stade A : (4R)-1-Méthyl-2-oxo-4-imidazolidine carboxylate de benzyle

Le produit attendu est obtenu selon le procédé décrit dans Synth. Commun. 1996, 26 (11), 2165-75.

### Stade B : (3R)-N-[(6-Amino-2-méthyl-3-pyridyl)-méthyl]-6-[(2,2-diphényléthyl) amino]-1-méthyl-5-oxo-1, 2 3,5-tétrahydroimidazo[1,2-a]pyrazine-3-carboxamide, dichlorhydrate

Le produit attendu est obtenu selon le procédé décrit dans l'exemple 1, à partir du composé obtenu dans le stade précédent, de 2,2-diphényléthylamine et du composé décrit dans la préparation A.

### EXEMPLE 95 : (8S)-N-[(6-Amino-2-méthyl-3-pyridyl)-méthyl]-2-oxo-3-(2-phénéthylamino)-1,4-diazabicyclo[4.2.0]octa-3,5-diène-8-carboxamide, dichlorhydrate

Le produit attendu est obtenu selon le procédé décrit dans l'exemple 1, à partir de (2*S*)-4-oxo-2-azétidine carboxylate de benzyle, de 2-phénéthylamine et du composé décrit dans la préparation A.

### EXEMPLE 96 : (6S)-N-[(6-Amino-2-méthyl-3-pyridyl)-méthyl]-4-oxo-3-(2-phénéthylamino)-4,6,7,8,9,10-hexahydropyrazino[1,2-a]azépine-6-carboxamide, dichlorhydrate

Le produit attendu est obtenu selon le procédé décrit dans l'exemple 1, à partir de (2*S*)-7-oxo-2-azépane carboxylate de benzyle, de 2-phénéthylamine et du composé décrit dans la préparation A.

### EXEMPLE 97 : (6R)-N-[(6-Amino-2-méthyl-3-pyridyl)-méthyl]-3-[2,2-bis-(4-méthoxyphényl)-éthylamino]-4-oxo-4,6,7,8-tétrahydropyrrolo[1,2-a]pyrazine-6-carboxamide, dichlorhydrate

Le produit attendu est obtenu selon le procédé décrit dans l'exemple 1, à partir de (2*R*)-5-oxoprolinate de benzyle, de 2,2-bis-(4-méthoxyphényl)-éthylamine et du composé décrit dans la préparation A.

### EXEMPLE 98 : N-[(6-Amino-2-méthyl-3-pyridyl)-méthyl]-3-[2,2-bis-(4-méthoxyphényl)-éthylamino]-4-oxo-4,6,7,8-tétrahydropyrrolo[1,2-a]pyrazine6-carboxamide, dichlorhydrate

Le produit attendu est obtenu selon le procédé décrit dans l'exemple 1, à partir de (±)-5-oxoprolinate de benzyle, de 2,2-bis-(4-méthoxyphényl)-éthylamine et du composé décrit dans la préparation A.

| *Microanalyse élémentaire* : | | | | |
|---|---|---|---|---|
| | *%C* | *%H* | *%N* | *%Cl* |
| *Calculé* | *59,33* | *5,78* | *13,39* | *11,30* |
| *Trouvé* | *60.19* | *5.70* | *13,50* | *11,61* |

### EXEMPLE 99 : (6S)-N-[(6-Amino-2-méthyl-3-pyridyl)-méthyl]-4-oxo-3-[(2R)-2-(5-méthyl-2-pyridyl)-2-(2,4-difluorophényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(5-méthyl-2-pyridyl)-2-(2,4-difluorophényl)-éthylamine et du composé décrit dans la préparation A.

### EXEMPLE 100 : (6S)-N-[(6-Amino-2-méthyl-3-pyridyl)-méthyl]-4-oxo-3-[(2S)-2-(5-méthyl-2-pyridyl)-2-(2,4-difluorophényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(5-méthyl-2-pyridyl)-2-(2,4-difluorophényl)-éthylamine et du composé décrit dans la préparation A.

### EXEMPLE 101 : (6S)-N-[(6-Amino-2-méthyl-3-pyridyl)-méthyl]-4-oxo-3-amino-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, d'imidodicarbonate de di-(tert-butyle) et du composé décrit dans la préparation A.

### EXEMPLE 102 : (4S)-N-[(6-Amino-2-méthyl-3-pyridyl)-méthyl]-1-méthyl-6-oxo-7-(2-phénéthylamino)-1,3,4,6-tétrahydro-2H-pyrazino[1,2-a] pyrimidine-4-carboxamide, dichlorhydrate

Le produit attendu est obtenu selon le procédé décrit dans l'exemple 1, à partir de (4*S*)-1-méthyl-2-oxo-hexahydro-4-pyrimidinecarboxylate de benzyle, de 2-phénéthylamine et du composé décrit dans la préparation A.

### EXEMPLE 103 : (6S)-N-[2-(Ethylcarbamoylméthoxy)-benzyl]-4-oxo-3-(2,2-diphényléthylamino)-4,6,7,8-tétrahydropyrrolo[1,2-a]pyrazine-6-carboxamide, chlorhydrate

Le produit attendu est obtenu selon le procédé décrit dans les stades F à I de l'exemple 1, à partir du composé décrit dans le stade E de l'exemple 1, de 2-diphényléthylamine et du composé décrit dans la préparation E.

| *Microanalyse élémentaire* : | | | | |
|---|---|---|---|---|
| | *%C* C | *%H* | *%N* | *%CI* |
| *Calculé* | *65, 83* | *6,03* | *11,63* | *5,89* |
| *Trouvé* | *65,82* | *6,00* | *11,57* | *5,64* |

### EXEMPLE 104 : (6S)-N-[(6-Amino-2-méthyI-3-pyridyl)-méthyl]-4-oxo-3-[(2-phényl-cyclopropyl)amino]-4,6,7,8-tétrahydropyrrolo[1,2-a]pyrazine-6-carboxamide, dichlorhydrate

Le produit attendu est obtenu selon le procédé décrit dans les stades F à I de l'exemple 1, à partir du composé décrit dans le stade E de l'exemple 1, de (2-phényl-cyclopropyl)amine et du composé décrit dans la préparation A.

### EXEMPLE 105 : (6S)-N-[(6-Amino-2-méthyl-3-pyridyl)-méthyl]-4-oxo-3-[(2,2-diphényl-cyclopropyl)amino]-4,6,7,8-tétrahydropyrrolo[1,2-a] pyrazine-6-carboxamide, dichlorhydrate

Le produit attendu est obtenu selon le procédé décrit dans les stades F à I de l'exemple 1, à partir du composé décrit dans le stade E de l'exemple 1, de (2,2-diphényl-cyclopropyl)amine et du composé décrit dans la préparation A.

### EXEMPLE 106 : (6S)-N-[(6-Amino-2-méthyl-3-pyridyl)-méthyl]-4-oxo-3-[(2R)-2-(2,4-diméthylphényl)-2-(5-éthyl-2-pyrimidinyl)éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(2,4-diméthylphényl)-2-(5-éthyl-2-pyrimidinyl)éthylamine et du composé décrit dans la préparation A.

### EXEMPLE 107 : (6S)-N-[(6-Amino-2-méthyl-3-pyridyl)-méthyl]-4-oxo-3-[(2S)-2-(2,4-diméthylphényl)-2-(5-éthyl-2-pyrimidinyl)éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(2,4-diméthylphényl)-2-(5-éthyl-2-pyrimidinyl)éthylamine et du composé décrit dans la préparation A.

### EXEMPLE 108 : (6S)-N-[(6-Amino-2-méthyl-3-pyridyl)-méthyl]-4-oxo-3-[(2R)-2-(5-méthyl-2-pyridyl)-2-(p-tolyl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(5-méthyl-2-pyridyl)-2-(p-tolyl)-éthylamine et du composé décrit dans la préparation A.

### EXEMPLE 109 : (6S)-N-[(6-Amino-2-méthyl-3-pyridyl)-méthyl]-4-oxo-3-[(2S)-2-(5-méthyl-2-pyridyl)-2-(p-tolyl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(5-méthyl-2-pyridyl)-2-(p-tolyl)-éthylamine et du composé décrit dans la préparation A.

### EXEMPLE 110 : (6S)-N-[(6-Amino-2-méthyl-3-pyridyl)-méthyl]-4-oxo-3-[(2R)-2-(5-méthoxy-2-pyridyl)-2-(4-méthoxyphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(5-méthoxy-2-pyridyl)-2-(4-méthoxyphényl)-éthylamine et du composé décrit dans la préparation A.

### EXEMPLE 111 : (6S)-N-[(6-Amino-2-méthyl-3-pyridyl)-méthyl]-4-oxo-3-[(2S)-2-(5-méthoxy-2-pyridyl)-2-(4-méthoxyphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(5-méthoxy-2-pyridyl)-2-(4-méthoxyphényl)-éthylamine et du composé décrit dans la préparation A.

### EXEMPLE 112 : (6S)-N-[(6-Amino-2-méthyl-3-pyridyl)-méthyl]-4-oxo-3-[(2R)-2-(5-méthoxy-2-pyridyl)-2-(3,4-diméthylphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*R*)-2-(5-méthoxy-2-pyridyl)-2-(3,4-diméthylphényl)-éthylamine et du composé décrit dans la préparation A.

### EXEMPLE 113 : (6S)-N-[(6-Amino-2-méthyl-3-pyridyl)-méthyl]-4-oxo-3-[(2S)-2-(5-méthoxy-2-pyridyl)-2-(3,4-diméthylphényl)-éthylamino]-4,6,7,8-tétrahydropyrrolo[1,2-a]pyrazine-6-carboxamide, trichlorhydrate

Le produit attendu est obtenu selon le procédé décrit dans les stades F à I de l'exemple 1, à partir du composé décrit dans le stade E de l'exemple 1, de (2*S*)-2-(5-méthoxy-2-pyridyl)-2-(3,4-diméthylphényl)-éthylamine et du composé décrit dans la préparation A.

Les exemples suivants ont été préparés selon le procédé décrit dans l'exemple 1 en utilisant les produits de départ correspondants

### EXEMPLE 114 : N-[(6-Amino-2-méthyl-3-pyridyl)-méthyl]-3-[(2,2-diphényléthyl)amino]-4-oxo-4,6,7,8-tétrahydropyrrolo[1,2-a]pyrazine-6-carboxamide, dichlorhydrate

| *Microanalyse élémentaire* | | | | |
|---|---|---|---|---|
| | *%C* | *%H* | *%N* | *% Cl* |
| *Calculé* | *61,38* | *5,68* | *14,81* | *12,19* |
| *Trouvé* | *61,9*7 | *3, 76* | *15,10* | *12,41* |

### EXEMPLE 115 : (6R)-N-[(6-Amino-2-méthyl-3-pyridyl)-méthyl]-3-[(2,2-diphényléthyl)amino]-4-oxo-4,6,7,8-tétrahydropyrrolo[1,2-a] pyrazine-6-carboxamide, dichlorhydrate

### EXEMPLE 116 : (6S)-N-[(6-Amino-2-méthyl-3-pyridyl)-méthyl]-4-oxo-3-[(2R)-2-(2-pyridyl)-2-(4-trifluorométhoxyphényl)-éthylamino]-4,6,7,8-tétrahydropyrrolo[1,2-a]pyrazine-6-carboxamide, trichlorhydrate

### EXEMPLE 117 : (6S)-N-[(6-Amino-2-méthyl-3-pyridyl)-méthyl]-4-oxo-3-[(2S)-2-(2-pyridyl)-2-(4-trifluorométhoxyphényl)-éthylamino]-4,6,7,8-tetrahydropyrrolo[1,2-a] pyrazine-6-carboxamide, trichlorhydrate

### EXEMPLE 118: (6S)-N-[(6-Amino-2-méthyl-3-pyridyl)-méthyl]-4-oxo-3-[(2R)-2-(4,6-diméthyl-2-pyridyl)-2-(4-méthoxyphényl)-éthylamino]-4,6,7,8-tétrahydropyrrolo[1,2-a]pyrazine-6-carboxamide, trichlorhydrate

### EXEMPLE 119: (6S)-N-[(6-Amino-2-méthyl-3-pyridyl)-méthyl]-4-oxo-3-[(2S)-2-(4,6-diméthyl-2-pyridyl)-2-(4-méthoxyphényl)-éthylamino]-4,6,7,8-tétrahydropyrrolo[1,2-a]pyrazine-6-carboxamide, trichlorhydrate

### EXEMPLE 120 : (6S)-N-[(6-Amino-2-méthyl-3-pyridyl)-méthyl]-4-oxo-3-[(2R)-2-(5-méthyl-2-pyridyl)-2-(2-fluoro-4-méthoxyphényl)-éthylamino]-4,6,7,8-tétrahydropyrrolo[1,2-a]pyrazine-6-carboxamide, trichlorhydrate

### EXEMPLE 121 : (6S)-N-[(6-Amino-2-méthyl-3-pyridyl)-méthyl]-4-oxo-3-[(2S)-2-(5-méthyl-2-pyridyl)-2-(2-fluoro-4-méthoxyphényl)-éthylamino]-4,6,7,8-tétrahydropyrrolo[1,2-a]pyrazine-6-carboxamide, trichlorhydrate

### EXEMPLE 122 : (6S)-N-[(6-Amino-2-méthyl-3-pyridyl)-méthyl]-4-oxo-3-[(2R)-2-(5-méthoxy-2-pyridyl)-2-(2,6-diméthyl-4-méthoxyphényl)-éthylamino]-4,6,7,8-tétrahydropyrrolo[1,2-a]pyrazine-6-carboxamide, trichlorhydrate

### EXEMPLE 123 : (6S)-N-[(6-Amino-2-méthyl-3-pyridyl)-méthyl]-4-oxo-3-[(2S)-2-(5-méthoxy-2-pyridyl)-2-(2,6-diméthyl-4-méthoxyphényl)-éthylamino]4,6,7,8-tétrahydropyrrolo[1,2-a]pyrazine-6-carboxamide, trichlorhydrate

### EXEMPLE 124 : (6S)-N-[(6-Amino-2-méthyl-3-pyridyl)-méthyl]-4-oxo-3-[(2R)-2-(4,6-diméthyl-2-pyridyl)-2-(4-méthyl-2-méthoxyphényl)-éthylamino]-4,6,7,8-tétrahydropyrrolo[1,2-a]pyrazine-6-carboxamide, trichlorhydrate

### EXEMPLE 125 : (6S)-N-[(6-Amino-2-méthyl-3-pyridyl)-méthyl]-4-oxo-3-[(2S)-2-(4,6-diméthyl-2-pyridyl)-2-(4-méthyl-2-méthoxyphényl)-éthylamino]-4,6,7,8-tétrahydropyrrolo[1,2-a]pyrazine-6-carboxamide, trichlorhydrate

### EXEMPLE 126 : (6S)-N-[(6-Amino-2-méthyl-3-pyridyl)-methyl]-3-[2,2-bis-(4-diméthylamino-phényl)-éthylamino]-4-oxo-4,6,7,8-tétrahydropyrrolo[1,2-a]pyrazine-6-carboxamide, tétrachlorhydrate

### EXEMPLE 127 : (6S)-N-[(6-Amino-2-méthyl-3-pyridyl)-méthyl]-3-[2,2-bis-(2-fluorophényl)-éthylamino]-4-oxo-4,6,7,8-tétrahydropyrrolo[1,2-a] pyrazine-6-carboxamide, dichlorhydrate

### EXEMPLE 128 : (6S)-N-[(6-Amino-2-méthyl-3-pyridyl)-méthyl]-3-[2,2-bis-(2-méthoxy-phényl)-éthylamino]-4-oxo-4,6,7,8-tétrahydropyrrolo [1,2-a]pyrazine-6-carboxamide, dichlorhydrate

### EXEMPLE 129 : (6S)-N-[(6-Amino-2-méthyl-3-pyridyl)-méthyl]-3-[2,2-bis-(2-chlorophényl)-éthylamino]-4-oxo-4,6,7,8-tétrahydropyrrolo[1,2-a] pyrazine-6-carboxamide, dichlorhydrate

### EXEMPLE 130 : (6S)-N-[(6-Amino-2-méthyl-3-pyridyl)-méthyl]-3-[2,2-bis-(2-hydroxy-phényl)-éthylamino]-4-oxo-4,6,7,8-tétrahydropyrrolo [1,2-a]pyrazine-6-carboxamide, dichlorhydrate

### EXEMPLE 131 : (6S)-N-[(6-Amino-2-méthyl-3-pyridyl)-méthyl]-3-[2,2-bis-(3-méthoxy-phényl)-éthylamino]-4-oxo-4,6,7,8-tétrahydropyrrolo [1,2-a]pyrazine-6-carboxamide, dichlorhydrate

### EXEMPLE 132 : (6S)-N-[(6-Amino-2-méthyl-3-pyridyl)-méthyl]-3-[2,2-bis-(3-fluorophényl)-éthylamino]-4-oxo-4,6,7,8-tétrahydropyrrolo[1,2-a] pyrazine-6-carboxamide, dichlorhydrate

### EXEMPLE 133 : (6S)-N-[(6-Amino-2-méthyl-3-pyridyl)-méthyl]-3-[2,2-bis-(3-chlorophényl)-éthylamino]-4-oxo-4,6,7,8-tétrahydropyrrolo[1,2-a] pyrazine-6-carboxamide, dichlorhydrate

### EXEMPLE 134 : (6S)-N-[(6-Amino-2-méthyl-3-pyridyl)-méthyl]-3-[2,2-bis-(3-hydroxy-phényl)-éthylamino]-4-oxo-4,6,7,8-tétrahydropyrrolo [1,2-a]pyrazine-6-carboxamide, dichlorhydrate

### EXEMPLE 135 : (6S)-N-[(6-Amino-2-méthyl-3-pyridyl)-méthyl]-4-oxo-3-[(2R)-2-(4-méthoxy-phényl)-2-(2-benzo[c]pyridyl)-éthylamino]-4,6,7,8-tétrahydropyrrolo[1,2-a]pyrazine-6-carboxamide, trichlorhydrate

### EXEMPLE 136 : (6S)-N-[(6-Amino-2-méthyl-3-pyridyl)-méthyl]-4-oxo-3-[(2S)-2-(4-méthoxy-phényl)-2-(2-benzo[c]pyridyl)-éthylamino]-4,6,7,8-tétrahydropyrrolo[1,2-a]pyrazine-6-carboxamide, trichlorhydrate

### EXEMPLE 137 : (6S)-N-[(6-Amino-2-méthyl-3-pyridyl)-méthyl]-3-[2,2-bis-(2-thiényl)-éthylamino]-4-oxo-4,6,7,8-tétrahydropyrrolo[1,2-a] pyrazine-6-carboxamide, dichlorhydrate

### EXEMPLE 138 : (6S)-N-[(6-Amino-2-méthyl-3-pyridyl)-méthyl]-4-oxo-3-[(2R)-2-phényl-2-phénéthylamino]-4,6,7,8-tétrahydropyrrolo[1,2-a] pyrazine-6-carboxamide, dichlorhydrate

### EXEMPLE 139 : (6S)-N-[(6-Amino-2-méthyl-3-pyridyl)-méthyl]-4-oxo-3-[(2S)-2-phényl-2-phénéthylamino]-4,6,7,8-tétrahydropyrrolo[1,2-a] pyrazine-6-carboxamide, dichlorhydrate

### EXEMPLE 140 : (6S)-N-[(6-Amino-2-méthyl-3-pyridyl)-méthyl]-4-oxo-3-[(2R)-2-hydroxy-2-phényl-2-phénéthylamino]-4,6,7,8-tétrahydropyrrolo [1,2-a]pyrazine-6-carboxamide, dichlorhydrate

### EXEMPLE 141 : (6S)-N-[(6-Amino-2-méthyl-3-pyridyl)-méthyl]-4-oxo-3-[(2S)-2-hydroxy-2-phényl-2-phénéthylamino]-4,6,7,8-tétrahydropyrrolo [1,2-a]pyrazine-6-carboxamide, dichlorhydrate

### EXEMPLE 142 : (6S)-N-[(6-Amino-2-méthyl-3-pyridyl)-méthyl]-4-oxo-3-[N-méthyl-2-phényl-2-phénéthylamino]-4,6,7,8-tétrahydropyrrolo[1,2-a] pyrazine-6-carboxamide, dichlorhydrate

### EXEMPLE 143 : (6S)-N-[(6-Amino-2-méthyl-3-pyridyl)-méthyl]-4-oxo-3-[N-méthyl-2-(2-pyridyl)-2-phénéthylamino]-4,6,7,8-tétrahydropyrrolo [1,2-a]pyrazine-6-carboxamide, trichlorhydrate

### EXEMPLE 144 : (6S)-N-[(6-Amino-2-méthyl-3-pyridoxyde)-méthyl]-3-[(2,2-diphényléthyl)amino]-4-oxo-4,6,7,8-tétrahydropyrrolo[1,2-a]pyrazine-6-carboxamide, dichlorhydrate

### EXEMPLE 145 : (6S)-N-[(4,6-Diméthyl-2-éthylaminocarbonylméthoxy-3-pyridyl)-méthyl]-3-[(2,2-diphényléthyl)amino]-4-oxo-4,6,7,8-tétrahydropyrrolo[1,2-a]pyrazine-6-carboxamide, dichlorhydrate

### EXEMPLE 146 : (6S)-N-[2-(Ethylcarbamoylméthoxy)-benzyl]-4-oxo-3-[(2R)-2-(5-méthyl-2-pyridyl)-2-(p-tolyl)éthylamino]-4,6,7,8-tétrahydropyrrolo[1,2-a]pyrazine-6-carboxamide, dichlorhydrate

### EXEMPLE 147 : (6S)-N-[2-(Ethylcarbamoylméthoxy)-benzyl]-4-oxo-3-[(2S)-2-(5-méthyl-2-pyridyl)-2-(p-tolyl)éthylamino]-4,6,7,8-tétrahydropyrrolo[1,2-a]pyrazine-6-carboxamide, dichlorhydrate

### EXEMPLE 148 : (6S)-N-[(6-Amino-2,5-diméthyl-3-pyridyl)-méthyl]-3-[(2R)-2-(5-méthyl-2-pyridyl)-2-(p-tolyl)-éthylamino]-4-oxo-4,6,7,8-tétrahydropyrrolo[1,2-a]pyrazine-6-carboxamide, trichlorhydrate

### EXEMPLE 149 : (6S)-N-[(6-Amino-2,5-diméthyl-3-pyridyl)-méthyl]-3-[(2S)-2-(5-méthyl-2-pyridyl)-2-(p-tolyl)-éthylamino]-4-oxo-4,6,7,8-tétrahydropyrrolo[1,2-a]pyrazine-6-carboxamide, trichlorhydrate

### EXEMPLE 150 : (6S)-N-[(6-Amino-2-méthyl-3-pyridyl)-méthyl]-4-oxo-3-[(2R)-2-(5-méthyl-2-pyridyl)-2-(4-méthyl-2-méthoxyphényl)-éthylamino]-4,6,7,8-tétrahydropyrrolo[1,2-a]pyrazine-6-carboxamide, trichlorhydrate

### EXEMPLE 151 : (6S)-N-[(6-Amino-2-méthyl-3-pyridyl)-méthyl]-4-oxo-3-[(2S)-2-(5-méthyl-2-pyridyl)-2-(4-méthyl-2-méthoxyphényl)-éthylamino]-4,6,7,8-tétrahydropyrrolo[1,2-a]pyrazine-6-carboxamide, trichlorhydrate

### EXEMPLE 152 : (6S)-N-[(6-Amino-2-méthyl-3-pyridyl)-méthyl]-4-oxo-3-[(2R)-2-(4,6-diméthyl-2-pyridyl)-2-(2,4-diméthoxyphényl)-éthylamino]-4,6,7,8-tétrahydropyrrolo[1,2-a]pyrazine-6-carboxamide, trichlorhydrate

### EXEMPLE 153 : (6S)-N-[(6-Amino-2-méthyl-pyridyl)-méthyl]-4-oxo-3-[(2S)-2-(4,6-diméthyl-2-pyridyl)-2-(2,4-diméthoxyphényl)-éthylamino]-4,6,7,8-tétrahydropyrrolo[1,2-a]pyrazine-6-carboxamide, trichlorhydrate

### ETUDE PHARMACOLOGIQUE DES DERIVES DE L'INVENTION

### EXEMPLE 154 : Activité anti-coagulante, mesure de temps de thrombine et temps de céphaline activée chez l'homme

Afin d'évaluer l'activité anti-coagulante des composés de l'invention, le temps de thrombine (TT) et le temps de céphaline activée (TCA) ont été déterminés dans des échantillons de plasma humain. Un coagulomètre ST₄ (Diagnostica Stago, France) a été utilisé. Un plasma, pauvre en plaquettes, lyophilisé (Stago), est repris dans de l'eau distillée. Le TT est réalisé avec le réactif Thrombine Prest et le TCA avec le réactif Céphaline PTT Automate.
L'inhibiteur ou le solvant (10 µl) est additionné au plasma (90 ul), puis incubé 2 minutes à 37°C. 100 µl de Thrombine Prest (TT) ou de Céphaline PTT Automate (TCA) sont ajoutés en déclenchant le chronomètre.

Dans ces conditions, le TT est de l'ordre de 18 secondes, le TCA est de l'ordre de 12 secondes. L'activité d'un antagoniste est évaluée par sa capacité de prolonger le TT et le TCA par rapport au témoin. L'effet des inhibiteurs est exprimé par la concentration en uM qui multiplie de 2 le temps de coagulation (Ctt₂).

Les composés de l'invention ont produit des prolongations des temps de coagulation très importantes et les Ctt₂ sont exemplifiés dans le tableau 1 ci-dessous:

**Tableau 1**

| *Exemple* | *TT Ctt*_{*2*} *(µM)* | *TCA Ctt*_{*2*} *(µM)* |
|---|---|---|
| 1 | 3 | 9,1 |
| 6 | 0,4 | 2,5 |
| 7 | 3 | 9,1 |
| 15 | 0,11 | 1,3 |
| 16 | 0,17 | 1,6 |
| 20 | 0,15 | 2,17 |
| 22 | 0,22 | 3,07 |
| 23 | 0,24 | 2,75 |
| 68 | 0,23 | 2,04 |
| 98 | 0,33 | 3,54 |
| 103 | 0,96 | 3,65 |

### EXEMPLE 155 : Inhibition de la thrombine et de protéases à sérine de la fibrinolyse

Pour évaluer in vitro l'activité inhibitrice des produits de l'invention sur la thrombine humaine (Sigma, activité spécifique 3230 UNIH/mg), le fibrinogène humain purifié (4 mM, Stago) (Fg) a été ajouté à une quantité donnée de thrombine (0.7 nM) préalablement incubée avec ou sans l'inhibiteur à tester (20°C, 10 minutes).

Pour évaluer in vitro la sélectivité de ces produits vis-à-vis de la plasmine, le même protocole a été appliqué à la plasmine humaine purifiée (2 nM, Stago), en utilisant pour substrat un peptide paranitroanilidé : <Glu-Phe-Lys-pNA (0.50 mM, S 2403, Kabi).

Inhibiteurs, enzymes et substrats sont dilués dans le même tampon (tampon phosphate 0.01 mM, pH 7.4, contenant 0.12 M de chlorure de sodium et 0.05 % de sérum albumine bovine) puis distribués dans une microplaque en polystyrène sous un volume de 50 µl.

La fibrine formée par la thrombine ou par le paranitroanilide libéré par l'action de la protéase à sérine est mesurée spectrophotométriquement à 405 nm après 15 à 30 minutes de réaction à 20°C.

Le tableau 2 ci-dessous donne la concentration des composés en nM inhibant 50 % de l'activité enzymatique (CI₅₀) de la thrombine par rapport au contrôle sans produit. Les résultats obtenus démontrent que les composés de l'invention sont des inhibiteurs puissants de la thrombine humaine vis-à-vis du fibrinogène humain.

**Tableau 2**

| *Exemple* | *CI*_{*50*} *(nM)* |
|---|---|
| 1 | 34 |
| 6 | 11 |
| 7 | 42 |
| 15 | 7 |
| 16 | 17 |
| 20 | 6 |
| 22 | 20 |
| 23 | 3,9 |
| 40 | 43 |
| 50 | 29 |
| 68 | 16 |
| 71 | 13 |
| 98 | 8 |
| 103 | 14 |

Le tableau 3 ci-dessous donne la concentration des composés en nM inhibant 50 % de l'activité enzymatique (CI₅₀) des protéases de la fibrinolyse. Les résultats obtenus démontrent que les composés de l'invention possèdent une sélectivité très importante vis-à-vis des protéases à sérine de la fibrinolyse.

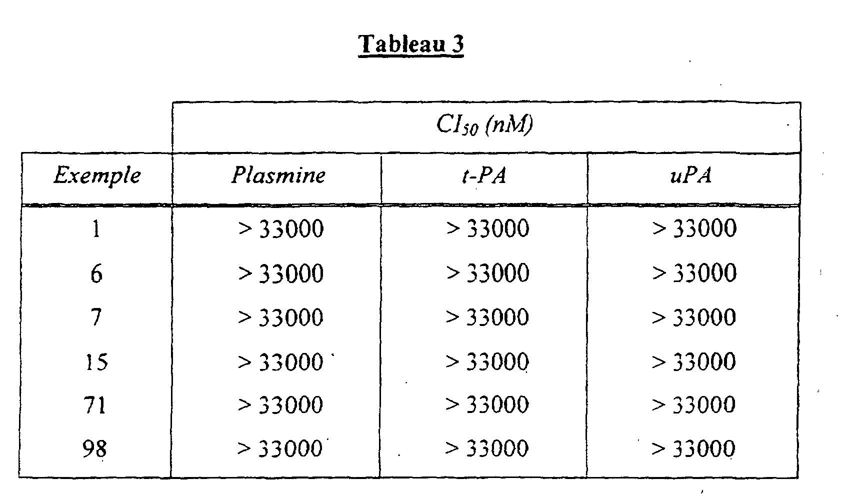

### EXEMPLE 156 : Activité anti-coagulante après administration per os chez le chien

Des chiens mâles ou femelles de 11-28 kg sont traités par voie orale avec les produits de l'invention (5 ou 10 mg/kg). Les temps de coagulation (TT, TCA) sont déterminés dans des échantillons de plasma des chiens 10 min avant et 30 min, 1 heure, 2 heures, 4 heures et 6 heures après l'administration des produits. Les mesures des temps de coagulation sont effectuées comme décrits dans l'exemple 114.

Dans les conditions de nos expériences, le TT est de l'ordre de 19 secondes et le TCA de l'ordre de 18 secondes.
Les substances de l'invention augmentent de façon importante les TT et les TCA chez les animaux. Le tableau 4 résume les résultats obtenus.
Les résultats montrent l'augmentation maximale des TT et TCA obtenue après traitement p.o. des chiens. Les valeurs montrent le nombre de fois que le temps initial est augmenté.

**Tableau 4**

| Augmentation de TT et de TCA (nombre de fois le temps initial) | | | |
|---|---|---|---|
| *Exemple* | *Dose (mg*/*kg)* | *TT* | *TCA* |
| 1 | 3 | 3,6 | 1,6 |
| 6 | 10 | 6,2 | 1,7 |
| 7 | 3 | 2,5 | 1,4 |
| 15 | 3 | 7 | 1,6 |
| 16 | 10 | 13,3 | 2,4 |
| 20 | 3 | 4,4 | 1,4 |
| 22 | 10 | 4,5 | 1,5 |
| 98 | 10 | 6,1 | 1,4 |

### EXEMPLE 157 : Composition pharmaceutique

Formule de préparation pour 1000 comprimés dosés à 10 mg :

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 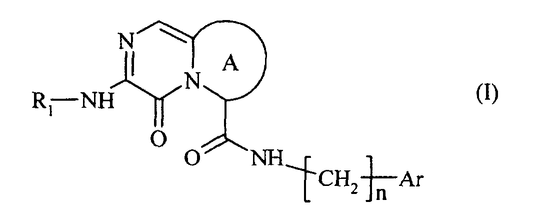 dans laquelle :
∗ R₁ représente un atome d'hydrogène ou un groupement alkyle (C₁-C₆) linéaire ou ramifié (éventuellement substitué par un ou plusieurs groupements, identiques ou différents, choisis parmi aryle, hétéroaryle, cycloalkyle, hétérocycloalkyle, carboxy, alkoxycarbonyle (C₁-C₆) linéaire ou ramifié, carbamoyle et hydroxy), ou un groupement cycloalkyle, un groupement hétérocycloalkyle ou un groupement de formule (G) : 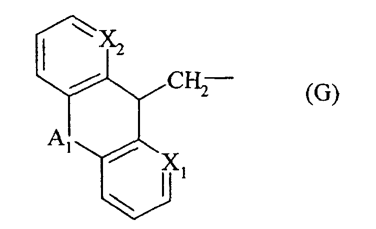 dans laquelle A₁ représente une liaison simple, un groupement -CH₂-, -CH₂-CH₂-, -N(CH₃)- ou un atome d'oxygène ou de soufre, et X₁ et X₂, identiques ou différents, représentent chacun un atome de carbone ou d'azote,
∗ 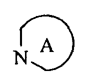 représente un cycle saturé de 4 à 7 chaînons pouvant contenir, en plus de l'atome d'azote, un ou deux hétéroatomes ou groupements choisis parmi O, S, ou -N(R₂),
• R₂ représente un atome d'hydrogène ou un groupement alkyle (C₁-C₆) linéaire ou ramifié,
∗ n représente un entier tel que 1≤n≤6,
∗ Ar représente un groupement aryle ou hétéroaryle,
leurs isomères, leurs N-oxydes ainsi que leurs sels d'addition à un acide ou à une base pharmaceutiquement acceptable,
étant entendu que par groupement aryle, on entend phényle, biphénylyle ou naphtyle, chacun de ces groupements étant éventuellement substitué par un ou plusieurs groupements, identiques ou différents, choisis parmi halogène, alkyle (C₁-C₆) linéaire ou ramifié (éventuellement substitué par un groupement hydroxy, carboxy ou carbamoyle (lui-même éventuellement substitué par un ou deux groupements alkyle (C₁-C₆) linéaire ou ramifié)), alkoxy (C₁-C₆) linéaire ou ramifié, hydroxy, trihalogénoalkyle (C₁-C₆) linéaire ou ramifié, trihalogénoalkoxy (C₁-C₆) linéaire ou ramifié, amino (éventuellement substitué par un ou deux groupements alkyle (C₁-C₆) linéaire ou ramifié), alkylcarbonyloxy (C₁-C₆) linéaire ou ramifié, carboxyméthoxy et carbamoylméthoxy (éventuellement N-substitué par un ou deux groupements alkyle (C₁-C₆) linéaire ou ramifié),
par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle (C₁-C₆) linéaire ou ramifié (éventuellement substitué par un groupement hydroxy, carboxy ou carbamoyle (lui-même éventuellement substitué par un ou deux groupements alkyle (C₁-C₆) linéaire ou ramifié)), hydroxy, alkoxy (C₁-C₆) linéaire ou ramifié, trihalogénoalkyle (C₁-C₆) linéaire ou ramifié, phényle, amino (éventuellement N-substitué par un ou plusieurs groupements alkyle (C₁-C₆) linéaire ou ramifié), carboxyméthoxy et carbamoylméthoxy (éventuellement N-substitué par un ou deux groupements alkyle (C₁-C₆) linéaire ou ramifié),
par groupement cycloalkyle, on entend un groupement hydrocarboné mono- ou bicyclique, saturé ou insaturé, de 3 à 12 chaînons, étant entendu que le cycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié) et aryle,
et par groupement hétérocycloalkyle, on entend un groupement mono- ou bicyclique, saturé ou insaturé, de 4 à 12 chaînons contenant un, deux ou trois hétéroatomes choisis parmi oxygène, azote ou soufre, étant entendu que l'hétérocycle peut être éventuellement substitué par un ou plusieurs groupements, identiques ou différents, choisis parmi halogène, alkyle (C₁-C₆) linéaire ou ramifié, alkoxy (C₁-C₆) linéaire ou ramifié, hydroxy, trihalogénoalkyle (C₁-C₆) linéaire ou ramifié, amino (substitué éventuellement par un ou plusieurs alkyle (C₁-C₆) linéaire ou ramifié), aryle et diarylméthyle.

2. Composé de formule (I) selon la revendication 1 tel que n est égal à 1

3. Composé de formule (I) selon la revendication 1 tel que 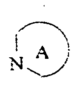 représente un groupement pyrrolidinyle.

4. Composé de formule (I) selon l'une quelconque des revendications 1 à 3 tel que Ar représente un groupement phényle ou pyridyle, chacun de ces groupements étant éventuellement substitué par un ou plusieurs groupements, identiques ou différents, choisis parmi halogène, alkyle (C₁-C₆) linéaire ou ramifié (éventuellement substitué par un groupement hydroxy, carboxy ou carbamoyle (lui-même éventuellement substitué par un ou deux groupements alkyle (C₁-C₆) linéaire ou ramifié)), alkoxy (C₁-C₆) linéaire ou ramifié, hydroxy, trihalogénoalkyle (C₁-C₆) linéaire ou ramifié, amino (éventuellement substitué par un ou deux groupements alkyle (C₁-C₆) linéaire ou ramifié), carboxyméthoxy et carbamoylméthoxy (éventuellement N-substitué par un ou deux groupements alkyle (C₁-C₆) linéaire ou ramifié).

5. Composé de formule (I) selon la revendication 4 tel que Ar représente un groupement pyridyle éventuellement substitué par un ou plusieurs groupements, identiques ou différents, choisis parmi halogène, alkyle (C₁-C₆) linéaire ou ramifié (éventuellement substitué par un groupement hydroxy, carboxy ou carbamoyle (lui-même éventuellement substitué par un ou deux groupements alkyle (C₁-C₆) linéaire ou ramifié)), alkoxy (C₁-C₆) linéaire ou ramifié, hydroxy, trihalogénoalkyle (C₁-C₆) linéaire ou ramifié, amino (éventuellement substitué par un ou deux groupements alkyle (C₁-C₆) linéaire ou ramifié), carboxyméthoxy et carbamoylméthoxy (éventuellement N-substitué par un ou deux groupements alkyle (C₁-C₆) linéaire ou ramifié).

6. Composé de formule (I) selon la revendication 1 qui est le (6S)-N-[(6-amino-2-méthyl-3-pyridyl)-méthyl]-4-oxo-3-(2-phénéthylamino)4,6,7,8-tétrahydropyrrolo[1,2-a] pyrazine-6-carboxamide, ses isomères ainsi que ses sels d'addition à un acide pharmaceutiquement acceptable.

7. Procédé de préparation des composés de formule (I) selon la revendication 1 **caractérisé en ce que** l'on réduit un composé de formule (II) : 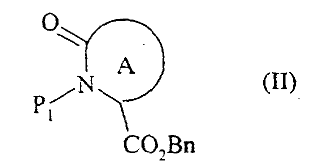 dans laquelle A a la même signification que dans la formule (I), P₁ représente un groupement protecteur de la fonction amino et Bn représente le groupement benzyle,
à l'aide d'un agent réducteur approprié,
pour conduire au composé de formule (III) : 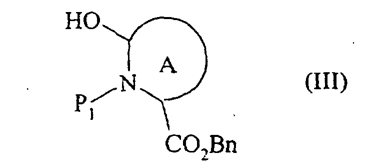 dans laquelle A, P₁ et Bn ont la même signification que précédemment,
composé de formule (III) dont on transforme la fonction hydroxy en méthoxy puis en fonction cyano par des réactions classiques de la chimie organique, pour conduire, après déprotection de la fonction amino, au composé de formule (IV) 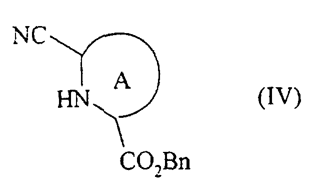 dans laquelle A et Bn ont la même signification que précédemment,
composé de formule (IV) que l'on met en réaction avec du chlorure d'oxalyle pour conduire au composé de formule (V) : 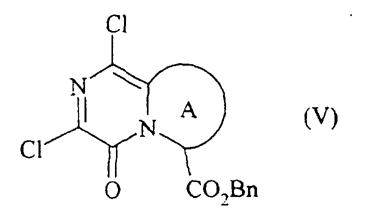 dans laquelle A et Bn ont la même signification que précédemment,
composé de formule (V) que l'on met en réaction avec un composé de formule (VI)
R₁-NH₂ (VI)
dans laquelle R₁ a la même signification que dans la formule (I), pour conduire au composé de formule (VII) : 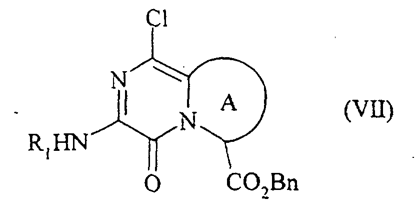 dans laquelle A, Bn et R₁ ont la même signification que précédemment,
composé de formule (VII) que l'on transforme ensuite par hydrogénation catalytique en composé de formule (VIII) : 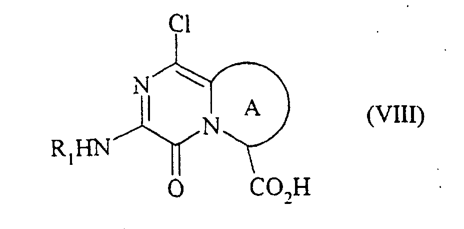 dans laquelle A et R₁ ont la même signification que précédemment,
composé de formule (VIII) que l'on transforme ensuite, par hydrogénation catalytique en milieu alcalin, en composé de formule (IX) 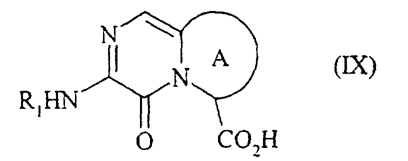 dans laquelle A et R₁ ont la même signification que précédemment,
composé de formule (IX) que l'on met en réaction avec un composé de formule (X) : 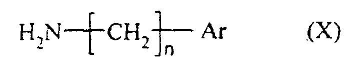 dans laquelle n et Ar ont la même signification que dans la formule (I),
pour conduire, après éventuelle déprotection, au composé de formule (I), composé de formule (I) que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

8. Composition pharmaceutique contenant comme principe actif un composé selon l'une quelconque des revendications 1 à 6, en combinaison avec un ou plusieurs véhicules inertes, non toxiques et pharmaceutiquement acceptables.

9. Composition pharmaceutique selon la revendication 8 utile en tant qu'inhibiteur de protéases à sérine apparentées à la trypsine.

10. Composition pharmaceutique selon la revendication 9 utile en tant qu'inhibiteur de thrombine.

## Patentansprüche

1. Verbindung der Formel (I): 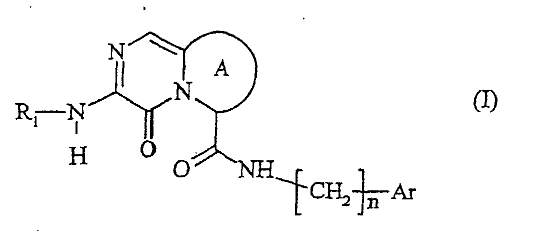 in der:
* R₁ ein Wasserstoffatom oder eine geradkettige oder verzweigte (C₁-C₆)-Alkylgruppe (die gegebenenfalls durch eine oder mehrere gleichartige oder verschiedene Gruppen ausgewählt aus Aryl, Heteroaryl, Cycloalkyl, Heterocycloalkyl, Carboxy, geradkettigem oder verzweigtem (C₁-C₆)-Alkoxycarbonyl, Carbamoyl und Hydroxy substituiert ist) oder eine Cycloalkylgruppe, eine Heterocycloalkylgruppe oder eine Gruppe der Formel (G): 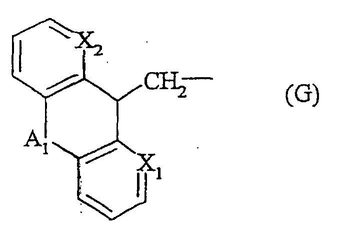 in der A₁ eine Einfachbindung, eine Gruppe -CH₂-, -CH₂-CH₂-, -N(CH₃)- oder ein Sauerstoff- oder ein Schwefelatom und X₁ und X₂, die gleichartig oder verschieden sind, jeweils ein Kohlenstoffatom oder ein Stickstoffatöm darstellen, bedeutet,
* 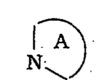 einen gesättigten Ring mit 4 bis 7 Kettengliedern darstellt, der neben dem Stickstoffatom ein oder zwei Heteroatome oder Gruppen ausgewählt aus 0, S und-N(R₂),
• worin R₂ ein Wasserstoffatom oder eine geradkettige oder verzweigte (C₁-C₆)-Alkylgruppe darstellt, enthalten kann,
* n eine ganze Zahl bedeutet, daß die Beziehung 1 ≤ n ≤ 6 erfüllt ist und
* Ar eine Aryl- oder Heteroarylgruppe bedeutet,
deren Isomere, N-Oxide sowie deren Additionssalze mit einer pharmazeutisch annehmbaren Säure oder Base,
mit der Maßgabe, daß unter einer Arylgruppe Phenyl, Biphenyl oder Naphthyl zu verstehen ist, wobei jede dieser Gruppen gegebenenfalls durch eine oder mehrere gleichartige oder verschiedene Gruppen ausgewählt aus Halogen, geradkettigem oder verzweigtem (C₁-C₆)-Alkyl (welches gegebenenfalls durch eine Hydroxy-, Carboxy- oder Carbamoylgruppe (die ihrerseits gegebenenfalls durch eine oder zwei geradkettige oder verzweigte (C₁-C₆)-Alkylgruppen substituiert sein kann) substituiert ist), geradkettigem oder verzweigtem (C₁-C₆)-Alkoxy, Hydroxy, geradkettigem oder verzweigtem (C₁-C₆)-Trihalogenalkyl, geradkettigem oder verzweigtem (C₁-C₆)-Trihalogenalkoxy, Amino (das gegebenenfalls durch eine oder zwei geradkettige oder verzweigte (C₁-C₆)-Alkylgruppen substituiert ist), geradkettigem oder verzweigtem (C₁-C₆)-Alkylcarbonyloxy, Carboxymethoxy und Carbamoylmethoxy (welches gegebenenfalls durch eine oder zwei geradkettige oder verzweigte (C₁-C₆)-Alkylgruppen N-substituiert ist) substituiert ist,
unter einer Heteroarylgruppe eine aromatische, mono- oder bicyclische Gruppe mit 5 bis 12 Kettengliedern zu verstehen ist, die ein, zwei oder drei Heteroatome ausgewählt aus Sauerstoff, Stickstoff oder Schwefel enthält, mit der Maßgabe, daß die Heteroarylgruppe gegebenenfalls durch eine oder mehrere, gleichartige oder verschiedenartige Gruppen substituiert sein kann ausgewählt aus Halogen, geradkettigem oder verzweigtem (C₁-C₆)-Alkyl (welches gegebenenfalls durch eine Hydroxy-, Carboxy- oder Carbamoylgruppe (welche ihrerseits gegebenenfalls durch eine oder zwei geradkettige oder verzweigte (C₁-C₆)-Alkylgruppen substituiert sein kann) substituiert ist), Hydroxy, geradkettigem oder verzweigtem (C₁-C₆)-Alkoxy, geradkettigem oder verzweigtem (C₁-C₆)-Trihalogenalkyl, Phenyl, Amino (welches gegebenenfalls durch eine oder mehrere geradkettige oder verzweigte (C₁-C₆)-Alkylgruppen N-substituiert ist), Carboxymethoxy und Carbamoylmethoxy (welches gegebenenfalls durch eine oder zwei geradkettige oder verzweigte (C₁-C₆)-Alkylgruppen N-substituiert ist),
unter einer Cycloalkylgruppe eine mono- oder bicyclische, gesättigte oder ungesättigte Kohlenwasserstoffgruppe mit 3 bis 12 Kettengliedern zu verstehen ist, wobei es sich versteht, daß der Ring gegebenenfalls durch eine oder mehrere gleichartige oder verschiedene Gruppen substituiert sein kann ausgewählt aus Halogen, geradkettigem oder verzweigtem (C₁-C₆)-Alkyl, geradkettigem oder verzweigtem (C₁-C₆)-Alkoxy, Hydroxy, geradkettigem oder verzweigtem (C₁-C₆)-Trihalogenalkyl, Amino (welches-gegebenenfalls durch eine oder mehrere geradkettige oder verzweigte (C₁-C₆)-Alkylgruppen substituiert sein kann) und Aryl,
und unter einer Heterocycloalkylgruppe eine mono- oder bicyclische, gesättigte oder ungesättigte Gruppe mit 4 bis 12 Kettengliedern zu verstehen ist, die ein, zwei oder drei Heteroatome ausgewählt aus Sauerstoff, Stickstoff und Schwefel enthält, wobei es sich versteht, daß der Heterocyclus gegebenenfalls durch eine oder mehrere gleichartige oder verschiedenartige Gruppen ausgewählt aus Halogen, geradkettigem oder verzweigtem (C₁-C₆)-Alkyl, geradkettigem oder verzweigtem (C₁-C₆)-Alkoxy, Hydroxy, geradkettigem oder verzweigtem (C₁-C₆)-Trihalogenalkyl, Amino (welches gegebenenfalls durch eine oder mehrere geradkettige oder verzweigte (C₁-C₆)-Alkylgruppen substituiert sein kann). Aryl und Diarylmethyl substituiert sein kann.

2. Verbindung der Forme (I) nach Anspruch 1, worin n den Wert 1 besitzt.

3. Verbindung der Formel (I) nach Anspruch 1, worin die Gruppe 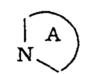 eine Pyrrolidinylgruppe bedeutet.

4. Verbindung der Formel (I) nach einem der Ansprüche 1 bis 3, worin Ar eine Phenyl- oder Pyridylgruppe darstellt, wobei jede dieser Gruppen gegebenenfalls durch eine oder mehrere gleichartige oder verschiedenartige Gruppen substituiert ist ausgewählt aus Halogen, geradkettigem oder verzweigtem (C₁-C₆)-Alkyl (welches gegebenenfalls durch eine Hydroxy-, Carboxy- oder Carbamoylgruppe (die ihrerseits gegebenenfalls durch eine oder zwei geradkettige oder verzweigte (C₁-C₆)-Alkylgruppen substituiert ist) substituiert ist), geradkettigem oder verzweigtem (C₁-C₆)-Alkoxy, Hydroxy, geradkettigem oder verzweigtem (C₁-C₆)-Trihalogenalkyl, Amino (welches gegebenenfalls durch eine oder zwei geradkettige oder verzweigte (C₁-C₆)-Alkylgruppen substituiert ist), Carboxymethoxy und Carbamoyhnethoxy (welches gegebenenfalls durch eine oder zwei geradkettige oder verzweigte (C₁-C₆)-AlkylgruppenN-substituiert ist).

5. Verbindung der Formel (I) nach Anspruch 4, worin Ar eine Pyridylgruppe bedeutet, die gegebenenfalls durch eine oder mehrere gleichartige oder verschiedene Gruppen substituiert ist ausgewählt aus Halogen, geradkettigem oder verzweigtem (C₁-C₆)-Alkyl (welches gegebenenfalls durch eine Hydroxy-, Carboxyoder Carbamoylgruppe (die ihrerseits gegebenenfalls durch eine oder zwei geradkettige oder verzweigte (C₁-C₆)-Alkylgruppen substituiert ist) substituiert ist), geradkettigem oder verzweigtem (C₁-C₆)-Alkoxy, Hydroxy, geradkettigem oder verzweigtem (C₁-C₆)-Trihalogenalkyl, Amino (welches gegebenenfalls durch eine oder zwei geradkettige oder verzweigte (C₁-C₆)-Alkylgruppen substituiert ist), Carboxymethoxy und Carbamoylmethoxy (welches gegebenenfalls durch eine oder zwei geradkettige oder verzweigte (C₁-C₆)-Alkylgruppen substituiert ist).

6. Verbindung der Formel (I) nach Anspruch 1, nämlich (6S)-N-[(6-Amino-2-methyl-3-pyridyl)-methyl]-4-oxo-3-(2-phenethylamino)-4,6,7,8-tetrahydropyrrolo-[1,2-a]pyrazin-6-carboxamid, dessen Isomere sowie dessen Additionssalze mit einer pharmazeutisch annehmbaren Säure.

7. Verfahren zur Herstellung der Verbindungen der Formel (I) nach Anspruch 1, **dadurch gekennzeichnet, daß** man eine Verbindung der Formel (II): 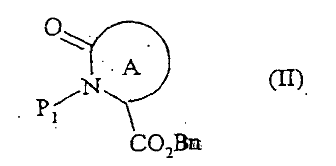 in der A die bezüglich der Formel (I) angegebenen Bedeutungen besitzt, P₁ eine Schutzgruppe für die Aminofunktion darstellt und Bn eine Benzylgruppe bedeutet,
mit Hilfe eines geeigneten Reduktionsmittels reduziert,
zur Bildung der Verbindung der Formel (III): 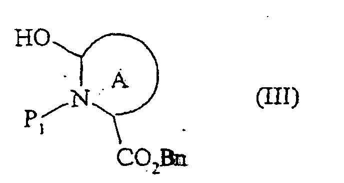 in der A, P₁ und Bn die oben angegebenen Bedeutungen besitzen,
bei welcher Verbindung der Formel (III) man die Hydroxyfunktion unter Anwendung klassischer Reaktionen, der organischen Chemie in eine Methoxygruppe und dann in eine Cyanofunktion umwandelt, so daß man nach der Abspaltung der Schutzgruppe der Aminofunktion die Verbindung der Formel (IV) erhält: 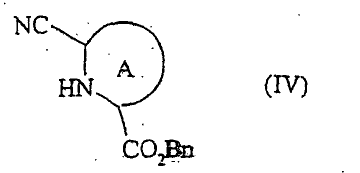 in der A und Bn die oben angegebenen Bedeutungen besitzen,
welche Verbindung der Formel (IV) man mit Oxalylchlorid umsetzt zur Bildung der Verbindung der Formel (V): 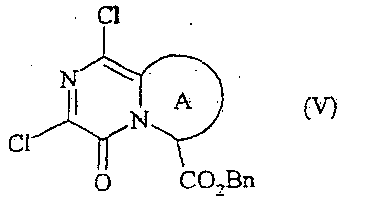 in der A und Bn die oben angegebenen Bedeutungen besitzen.
welche Verbindung der Formel (V) man mit einer Verbindung der Formel (VI):
R₁ - NH₂ (VI)
in der R₁ die bezüglich der Formel (I) angegebenen Bedeutungen besitzt, umsetzt zur Bildung der Verbindung der Formel (VII): 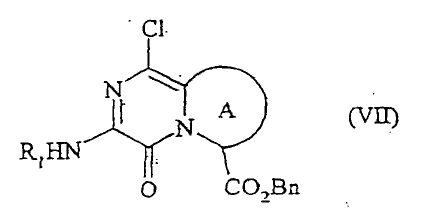 in der A, Bn und R₁ die oben angegebenen Bedeutungen besitzen,
welche Verbindung der Formel (VII) man anschließend durch katalytische Hydrierung in die Verbindung der Formel (VIII) umwandelt: 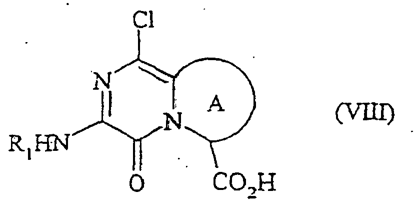 in der A und R₁ die oben angegebenen Bedeutungen besitzen,
welche Verbindung der Formel (VIII) man anschließend durch katalytische Hydrierung in alkalischem Medium in die Verbindung der Formel (IX) umwandelt: 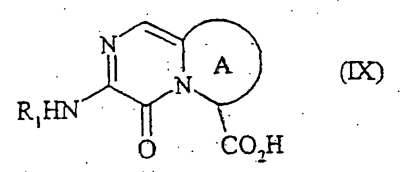 in der A und R₁ die oben angegebenen Bedeutungen besitzen,
welche Verbindung der Formel (IX) man mit einer Verbindung der Formel (X): 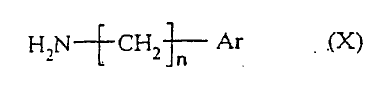 in der n und Ar die bezüglich der Formel (I) angegebenen Bedeutungen besitzen, umsetzt,
so daß man nach der eventuellen Abspaltung der Schutzgruppen die Verbindung der Formel (I) erhält,
welche Verbindung der Formel (I) man gegebenenfalls mit Hilfe einer klassischen Reinigungsmethode reinigt man gewünschtenfalls mit Hilfe einer klassischen Trennmethode in ihre Isomeren auftrennt und gewünschtenfalls mit einer pharmazeutisch annehmbaren Säure oder Base in ihre Additionssalze umwandelt.

8. Pharmazeutische Zubereitung, enthaltend als Wirkstoff eine Verbindung nach einem der Ansprüche 1 bis 6 in Kombination mit einem oder mehreren inerten, nichttoxischen und pharmazeutisch annehmbaren Trägermaterialien.

9. Pharmazeutische Zubereitung nach Anspruch 8, nützlich als Inhibitor von mit Trypsin verwandten Serinproteasen.

10. Pharmazeutische Zubereitung nach Anspruch 9, geeignet als Thrombininhibitor.

## Claims

1. Compound of formula (I): 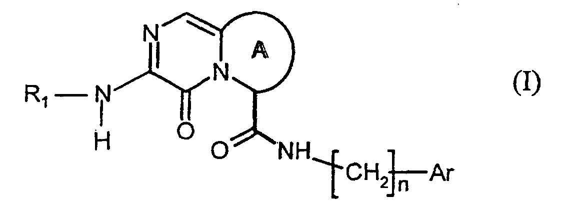 wherein :
∗ R₁ represents a hydrogen atom or a linear or branched (C₁-C₆)alkyl group (optionally substituted by one or more identical or different groups selected from aryl, heteroaryl, cycloalkyl, heterocycloalkyl, carboxy, linear or branched (C₁-C₆)alkoxycarbonyl, carbamoyl and hydroxy), or a cycloalkyl group, a heterocycloalkyl group or a group of formula (G) : 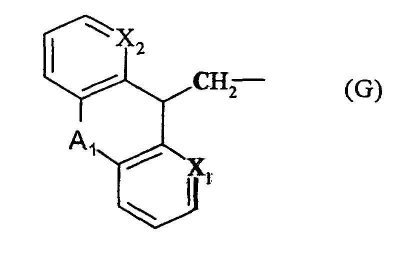
wherein A₁ represents a single bond, a -CH₂-, -CH₂-CH₂- or -N(CH₃)- group or an oxygen or sulphur atom, and X₁ and X₂, which may be identical or different, each represents a carbon or nitrogen atom,
∗ 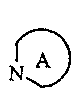 represents a saturated ring having from 4 to 7 ring members that may contain in addition to the nitrogen atom one or two hetero atoms or groups selected from O, S, and -N(R₂),
• R₂ represents a hydrogen atom or a linear or branched (C₁-C₆)alkyl group,
∗ n represents an integer wherein 1≤n≤6,
∗ Ar represents an aryl or heteroaryl group,
their isomers, their N-oxides and addition salts thereof with a pharmaceutically acceptable acid or base,
"aryl group" being understood to mean phenyl, biphenylyl or naphthyl, each of those groups being optionally substituted by one or more identical or different groups selected from halogen, linear or branched (C₁-C₆)alkyl (optionally substituted by a hydroxy or carboxy group or by a carbamoyl group (itself optionally substituted by one or two linear or branched (C₁-C₆)alkyl groups)), linear or branched (C₁-C₆)alkoxy, hydroxy, trihalo-(C₁-C₆)alkyl in which the alkyl moiety is linear or branched, trihalo-(C₁-C₆)alkoxy in which the alkoxy moiety is linear or branched, amino (optionally substituted by one or two linear or branched (C₁-C₆)alkyl groups), linear or branched (C₁-C₆)alkylcarbonyloxy, carboxymethoxy and carbamoylmethoxy (optionally N-substituted by one or two linear or branched (C₁-C₆)alkyl groups),
"heteroaryl group" being understood to mean a mono- or bi-cyclic aromatic group having from 5 to 12 ring members and containing one, two or three hetero atoms selected from oxygen, nitrogen and sulphur, it being understood that the heteroaryl may be optionally substituted by one or more identical or different groups selected from halogen, linear or branched (C₁-C₆)alkyl (optionally substituted by a hydroxy or carboxy group or by a carbamoyl group (itself optionally substituted by one or two linear or branched (C₁-C₆)alkyl groups)), hydroxy, linear or branched (C₁-C₆)alkoxy, trihalo-(C₁-C₆)alkyl in which the alkyl moiety is linear or branched, phenyl, amino (optionally N-substituted by one or more linear or branched (C₁-C₆)alkyl groups), carboxymethoxy and carbamoylmethoxy (optionally N-substituted by one or two linear or branched (C₁-C₆)alkyl groups), "cycloalkyl group" being understood to mean a saturated or unsaturated mono- or bi-cyclic hydrocarbon group having from 3 to 12 ring members, it being understood that the ring system may be optionally substituted by one or more identical or different groups selected from halogen, linear or branched (C₁-C₆)alkyl, linear or branched (C₁-C₆)alkoxy, hydroxy, trihalo-(C₁-C₆)alkyl in which the alkyl moiety is linear or branched, amino (optionally substituted by one or more linear or branched (C₁-C₆)alkyl groups) and aryl, and "heterocycloalkyl group" being understood to mean a saturated or unsaturated mono- or bi-cyclic group having from 4 to 12 ring members and containing one, two or three hetero atoms selected from oxygen, nitrogen and sulphur, it being understood that the heterocycle may be optionally substituted by one or more identical or different groups selected from halogen, linear or branched (C₁-C₆)alkyl, linear or branched (C₁-C₆)alkoxy, hydroxy, trihalo-(C₁-C₆)alkyl in which the alkyl moiety is linear or branched, amino (optionally substituted by one or more linear or branched (C₁-C₆)alkyl groups), aryl and diarylmethyl.

2. Compound of formula (I) according to claim 1 wherein n is 1.

3. Compound of formula (I) according to claim 1 wherein 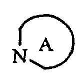 represents a pyrrolidinyl group.

4. Compound of formula (I) according to any one of claims 1 to 3 wherein Ar represents a phenyl or pyridyl group, each of those groups being optionally substituted by one or more identical or different groups selected from halogen, linear or branched (C₁-C₆)-alkyl (optionally substituted by a hydroxy or carboxy group or by a carbamoyl group (itself optionally substituted by one or two linear or branched (C₁-C₆)alkyl groups)), linear or branched (C₁-C₆)alkoxy, hydroxy, trihalo-(C₁-C₆)alkyl in which the alkyl moiety is linear or branched, amino (optionally substituted by one or two linear or branched (C₁-C₆)alkyl groups), carboxymethoxy and carbamoylmethoxy (optionally N-substituted by one or two linear or branched (C₁-C₆)alkyl groups).

5. Compound of formula (I) according to claim 4 wherein Ar represents a pyridyl group optionally substituted by one or more identical or different groups selected from halogen, linear or branched (C₁-C₆)alkyl (optionally substituted by a hydroxy or carboxy group or by a carbamoyl group (itself optionally substituted by one or two linear or branched (C₁-C₆)alkyl groups)), linear or branched (C₁-C₆)alkoxy, hydroxy, trihalo-(C₁-C₆)alkyl in which the alkyl moiety is linear or branched, amino (optionally substituted by one or two linear or branched (C₁-C₆)alkyl groups), carboxymethoxy and carbamoylmethoxy (optionally N-substituted by one or two linear or branched (C₁-C₆)alkyl groups).

6. Compound of formula (I) according to claim 1 which is (6S)-N-[(6-amino-2-methyl-3-pyridyl)methyl]-4-oxo-3-(2-phenethylamino)-4,6,7,8-tetrahydropyrrolo[1,2-a]-pyrazine-6-carboxamide, its isomers and its addition salts with a pharmaceutically acceptable acid.

7. Process for the preparation of compounds of formula (I) according to claim 1, **characterised in that** a compound of formula (II): 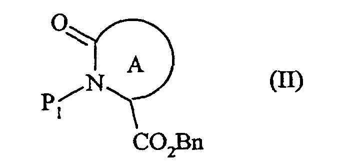 wherein A is as defined for formula (I), P₁ represents an amino-function-protecting group and Bn represents a benzyl group,
is reduced using an appropriate reducing agent,
to yield a compound of formula (III): 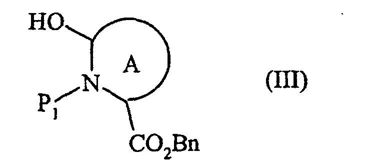 wherein A, P₁ and Bn are as defined hereinbefore,
the hydroxy function of which compound of formula (III) is converted to methoxy and then to the cyano function by conventional organic chemistry reactions, to yield, after deprotection of the amino function, a compound of formula (IV): 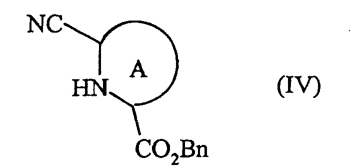 wherein A and Bn are as defined hereinbefore,
which compound of formula (IV) is reacted with oxalyl chloride to yield a compound of formula (V): 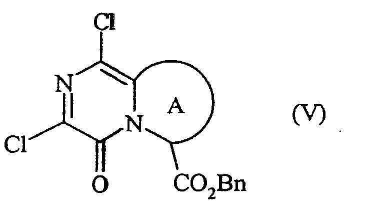 wherein A and Bn are as defined hereinbefore,
which compound of formula (V) is reacted with a compound of formula (VI) :
R₁-NH₂ (VI)
wherein R₁ is as defined for formula (I), to yield a compound of formula (VII) : 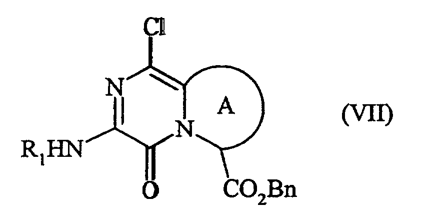 wherein A, Bn and R₁ are as defined hereinbefore,
which compound of formula (VII) is then converted by catalytic hydrogenation to a compound of formula (VIII): 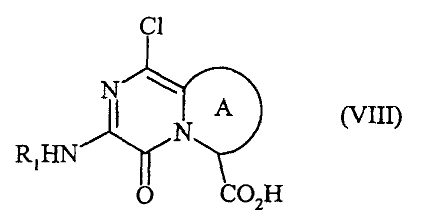 wherein A and R₁ are as defined hereinbefore,
which compound of formula (VIII) is then converted by catalytic hydrogenation in an alkaline medium to a compound of formula (Ix): 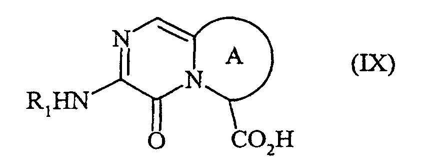 wherein A and R₁ are as defined hereinbefore,
which compound of formula (IX) is reacted with a compound of formula (X) : 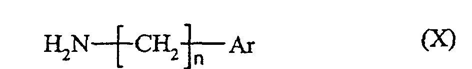 wherein n and Ar are as defined for formula (I),
to yield, after deprotection where appropriate, a compound of formula (I),
which compound of formula (I) is purified, if necessary, according to a conventional purification technique, separated, if desired, into its isomers according to a conventional separation technique, and converted, if desired, into its addition salts with a pharmaceutically acceptable acid or base.

8. Pharmaceutical composition comprising as active ingredient a compound according to any one of claims 1 to 6 in combination with one or more inert, non-toxic pharmaceutically acceptable carriers.

9. Pharmaceutical composition according to claim 8 for use as an inhibitor of trypsin-related serine proteases.

10. Pharmaceutical composition according to claim 9 for use as a thrombin inhibitor.
